# EUROPÄISCHE PATENTANMELDUNG

(11) **EP 1 273 904 A2**
(43) Veröffentlichungstag der Anmeldung: **08.01.2003**
(21) Anmeldenummer: 02013767.5
(22) Anmeldetag: 20.06.2002
(51) Int. Cl.: G01N 21/47, G01N 21/31, G01N 21/49

(54) **Verfahren und Vorrichtung zur Erfassung von Stoffen in vitalem Gewebe**

(30) Priorität: 20.06.2001 DE 10129754
(71) Anmelder: MBR GmbH, D-58313 Herdecke (DE)
(72) Erfinder: Jungmann, Holger, Dr., 45896 Gelsenkirchen (DE); Schietzel, Michael, Dr., 58313 Herdecke (DE); Schmidt, Martin, Dr., 87448 Waltenhofen (DE)
(74) Vertreter: Rössig, Rolf

(57) **Zusammenfassung**

Die Erfindung betrifft ein Verfahren und eine Vorrichtung zum Nachweis insbesondere zur Konzentrationsbestimmung von Stoffen in vitalem Gewebe. Der Erfindung liegt die Aufgabe zugrunde, ein Verfahren und eine Vorrichtung zur Erfassung von Stoffen in vitalem Gewebe zu schaffen, wodurch eine zuverlässige qualitative und quantitative Detektion dieser Stoffe auf physiologisch unbedenkliche Weise ermöglicht wird. Diese Aufgabe wird erfindungsgemäß gelöst durch eine Verfahren zur Erfassung von Stoffen in vitalem Gewebe, bei welchem Licht mit einer vorgegebenen Wellenlänge derart auf das Gewebe gerichtet wird, dass das Licht in das Gewebe eindringt, zumindest ein Teil des aus dem Gewebe austretenden Lichtes erfasst wird und das remittierte Licht unter Zuordnung seiner Wellenlänge zur Intensität ermittelt wird, und die derart ermittelten Eigenschaften des remittierten Lichtes mit wenigstens einem Referenzsystem verglichen werden, wobei auf Grundlage einer Korrelation mit dem Referenzsystem auf die Präsenz und/oder Konzentration eines Stoffes geschlossen wird.

## Beschreibung

Die Erfindung betrifft ein Verfahren und eine Vorrichtung zum Nachweis insbesondere zur Konzentrationsbestimmung von Stoffen in vitalem Gewebe.

Der Erfindung liegt die Aufgabe zugrunde, ein Verfahren und eine Vorrichtung zur Erfassung von Stoffen in vitalem Gewebe zu schaffen, wodurch eine zuverlässige qualitative und quantitative Detektion dieser Stoffe auf physiologisch unbedenkliche Weise ermöglicht wird.

Diese Aufgabe wird erfindungsgemäß gelöst durch ein Verfahren zur Erfassung von Stoffen in vitalem Gewebe, bei welchem Licht mit einer vorgegebenen Wellenlänge derart auf das Gewebe gerichtet wird, dass das Licht in das Gewebe eindringt, zumindest ein Teil des aus dem Gewebe austretenden Lichtes erfasst wird, und die Intensität des remittierten Lichtes, oder die optische Dichte des beleuchteten Mediums unter Zuordnung zu seiner Wellenlänge ermittelt wird und die derart ermittelte Intensitätsverteilung des remittierten Lichtes oder der optischen Dichte, mit wenigstens einem Referenzsystem verglichen wird, wobei auf Grundlage des Vergleichsergebnisses auf die Präsenz und/oder Konzentration eines Stoffes geschlossen wird.

Dadurch wird es auf vorteilhafte Weise möglich, verschiedene Substanzen in-vivo, nicht-invasiv zu erfassen und etwaige medizinische Hilfs- oder Therapiemaßnahmen präzise auf den physiologischen Zustand dieser Person abzustimmen. In besonders vorteilhafter Weise wird es auch möglich, krankheitsbedingt im Kreislauf der Person befindliche Substanzen zu detektieren und hierauf basierend eine Medikation abzustimmen. In weiterhin vorteilhafter Weise wird es möglich, psychoaktive Substanzen, deren Abbauprodukte, illegale Drogen sowie Pharmaka und ebenfalls deren Metabolite zu erfassen.

Gemäß einer besonders bevorzugten Ausführungsform des Verfahrens sind die Referenzen beispielsweise in Form von datensatzartig spezifizierten Referenzspektren derart abgestimmt, daß diese - oder die Korrelationen hiermit - beispielsweise in Form von Übereinstimmungen/Abweichungen hiervon - jeweils für einen bestimmten Stoff - oder eine Stoffgruppe indikativ sind.

Alternativ hierzu - oder in besonders vorteilhafter Weise auch in Kombination mit der vorangehend genannten Maßnahme - ist es auch möglich, mehrere Referenzsysteme insbesondere eine Vielzahl von datensatzartig bereitgehaltenen, z.B. abgespeicherten Referenzspektren vorzusehen, wobei in Abhängigkeit von der Erfüllung vorgegebener Beziehungen mit den Referenzsystemen, insbesondere Referenzspektren und der erfassten Spektralverteilung auf die Präsenz und/oder Konzentration eines Stoffes geschlossen wird.

In vorteilhafter Weise wird auf Grundlage der Intensität ausgewählter Wellenlängenbereiche, ein Stoff identifiziert und auf die Konzentration des erkannten Stoffes geschlossen. Diese Wellenlängenbereiche werden gemäß einer besonders bevorzugten Ausführungsform des erfindungsgemäßen Verfahrens mit Blick auf ein erwartetes Klassifikationsergebnis ausgewählt.

Das Spektrum des auf das Gewebe abgestrahlten Lichtes erstreckt sich vorzugsweise über einen Wellenlängenbereich von 200 bis 800nm. Zum Nachweis psychoaktiver Substanzen eignet sich insbesondere ein Wellenlängenfenster zwischen 220 bis 400nm.

Es ist möglich, die spektrale Breite des auf das Gewebe abgestrahlten Lichtes auf einen Wert festzulegen, der kleiner ist als die Bandbreite des Analysebereiches beispielsweise 60nm. Insbesondere ist es möglich, im wesentlichen monochromes, insbesondere kohärentes Licht zu verwenden und dessen Frequenz sukzessive über den Analysebereich beispielsweise von 220 400nm zu modulieren. Die Modulation erfolgt vorzugsweise derart, daß die Wellenlänge des Lichtes in Schritten von 0,3 nm sukzessive verändert wird. Bei der Verwendung von monochromem Licht wird es möglich, Fluoreszenz- oder Bandverschiebungseffekte, welche durch die gesuchten Substanz hervorgerufen werden, beim Nachweis derselben zu nutzen. Alternativ zu der Verwendung von monochromem Licht ist es auch möglich bei diesem Vorgehen weißes Licht zu verwenden.

Gemäß einer besonders bevorzugten Ausführungsform der Erfindung erfolgt der Vergleich der erfassten Wellenlängen/Instensitäts-Verteilung auf Grundlage einer Korrelationsbetrachtung. In Abhängigkeit von der Erfüllung einer Korrelationsbeziehung kann hierbei auf die Präsenz und/oder Konzentration eines Stoffes, oder von Stoffgruppen geschlossen werden.

Vorzugsweise sind mehrere, zur Identifikation von Amphetaminen, Benzodiazepinen, Cannabinoiden, Methadon, Antiepileptika und Ecgoninen herangezogene Referenzgrundlagen vorgesehen.

Weiterhin umfassen die Referenzgrundlagen vorzugsweise auch Datensätze zur Identifikation von Heroin-Derivaten, Kokain, LSD, Nor-LSD, Opiaten, Buprenorphin, Gabapentin, Carbamazepin, Oxcarbazepin, Antidepressiva, Neuroleptika, Barbiturate und Antibiotika.

Gemäß einer besonders bevorzugten Ausführungsform der Erfindung wird das Remissionslicht an Körperstellen mit unterschiedlichem Blutoxygenierungsgrad gemessen. Dadurch wird es möglich, den Sauerstoffgehalt des Blutes und den Hämoglobingehalt des Blutes zu bestimmen und Einflüsse des Blutoxygenierungsgrades bei der Signalauswertung zu berücksichtigen.

Das Referenzsystem stellt vorzugsweise zur Identifikation von Amphetaminen vorgesehene Referenzdaten bereit. In besonders vorteilhafter Weise stellt das Referenzsystem mehrere zur Identifikation von Benzodiazepinen vorgesehene Referenzdaten bereit. In weiterhin vorteilhafter Weise stellt das Referenzsystem mehrere zur Identifikation von Methadon-Enatiomeren und deren racemischem Gemisch bereit. In weiterhin vorteilhafter Weise stellt das Referenzsystem mehrere Referenzgrundlagen zur Identifikation von Heroin bereit. In weiterhin vorteilhafter Ausgestaltung stellt das Referenzsystem mehrere zur Identifikation von Buprenorphin, Gabapentin, Carbamazepin, Zolpidem, Zopiclon, Dextromethorphan, Doxepin, Promethazin und/oder Oxcarbazepin geeignete Referenzgrundlagen bereit. Das Referenzsystem stellt vorzugsweise auch mehrere Referenzgrundlagen zur Identifikation von Kokain und Ecgoninen bereit. Das Referenzsystem kann stellt in vorteilhafter Weise auch mehrere Referenzgrundlagen zur Identifikation von LSD und Nor-LSD bereit. Das Referenzsystem stellt vorzugsweise auch mehrere Referenzgrundlagen zur Identifikation von Opiaten bereit. Das Referenzsystem stellt vorzugsweise auch mehrere Referenzgrundlagen zur Identifikation von Cannabinoiden bereit. Vorzugsweise sind durch das Referenzsystem insbesondere mehrere Referenzgrundlagen zur Identifikation von Metaboliten der vorgenannten Substanzen bereitgestellt. Die Erfassung des Remissionslichtes wird vorzugsweise an wenigstens zwei örtlich verschiedenen Messstellen oder hinsichtlich Kapillarisationsmerkmalen, oder der Blutoxygenierung unterschiedlichen Messstellen vorgenommen. Vorzugsweise wird das Verfahren derart durchgeführt, dass die Abhängigkeit des Remissionsspektrums vom Oxygenierungsgrad Eingang in eine Signalauswertung findet.

In vorrichtungstechnischer. Hinsicht wird die eingangs angegebene Aufgabe auch gelöst durch eine Vorrichtung zur Erfassung von Stoffen in vitalem Gewebe, mit einer Lichtquelle zur Erzeugung von Licht mit einem vorgegebenen Spektrum und Aufstrahlung des Lichtes auf das vitale Gewebe derart, dass das Licht in das Gewebe eindringt, einer Lichterfassungseinrichtung zur Erfassung zumindest eines Teiles des aus dem Gewebe remittierten Lichtes, einer Erfassungseinrichtung zur Erfassung der Intensität des remittierten Lichtes unter Zuordnung zur Wellenlänge und einer Vergleichseinrichtung zum Vergleichen der ermittelten Intensitätsverteilung des Remissions-Spektrums mit wenigstens einem Referenzdatensatz, wobei auf Grundlage des Vergleichsergebnisses auf die Präsenz und/oder Konzentration eines Stoffes bzw. Stoffgruppe geschlossen wird.

In besonders vorteilhafter Weise umfaßt die Vorrichtung ein Auswertungssystem, das derart ausgebildet ist, daß dieses den Algorithmus, die Auswerteprozedur und/oder das Referenzsystem unter Einbezug der erfaßten Meßdaten adaptiv verändert. In vorteilhafter Weise ist die Vorrichtung derart ausgebildet, daß auf Grundlage der erfaßten Meßdaten eine Selbsteichung erfolgt. In vorteilhafter Weise ist die Vorrichtung derart ausgebildet, daß auf Grundlage der erfaßten Meßdäten eine Funktionskontrolle erfolgt. In vorteilhafter Weise ist die Vorrichtung derart ausgebildet, daß Wahrscheinlichkeitsaussagen über das Vorhandensein ggf. verwechselbarer Substanzen erfolgen. In weiterhin vorteilhafter Weise ist die Vorrichtung derart ausgebildet, daß eine automatische. Fehleranalyse bei jedem Meßvorgang erfolgt. In vorteilhafter Weise ist die Vorrichtung derart ausgebildet, daß eine automatische Fehleranalyse erfolgt, die beispielsweise die Wahrscheinlichkeit für zukünftige mögliche Meßfehler oder falsche Stoffideritifizierung minimiert.

In besonders vorteilhafter Weise werden die zu detektierende Substanzen durch Fluoreszenzeffekte identifizierbar. Licht, das vorzugsweise in einem Wellenlängenbereich von 200 bis 400 nm auf das zu untersuchende Gewebe in vivo aufgestrahlt wird, reichert die zu untersuchenden Stoffe energetisch an. In einem, gewissen, im Einzelfall auch stoffspezifischen zeitlichen Abstand geben die Stoffe einen Teil dieser Energie auf versetzten, insbesondere höheren Wellenlängenbereichen zwischen 240 und 1000 nm wieder ab. Anreicherungsenergie und Abstrahlungsenergie haben verschiedene Wellenlängenbereiche . Aus diesem Grunde können Anreicherungsenergie und Abstrahlungsenergie unterschieden werden. Die Abstrahlungsenergie ist geriner als die Anreicherungsenergie. Daraus ergibt sich, dass das Abstrahlungsspektrum (=Fluoreszenzspektrum) in einem höheren Wellenlängenbereich liegt. Diese energieärmere Abstrahlungsenergie ergibt das sog. Fluoreszenzspektrum und kann mittels einer Erfassungseinrichtung erfasst werden.

Die dergestallt erstellten Spektren ergeben charakteristische Signale in einem Wellenlängenbereich zwischen 240 und 1000 nm. Die Stoffe unterscheiden sich innerhalb des Spektrums durch charakteristische Abschnitte.Insbesondere die verknüpfte Betrachtung ausgewählter Abschnitte des Spektralbereiches des Fluoreszensspektrums ermöglicht auch bei hoher Überlagerung des Spektrums eine hinreichend zuverlässige Identifizierung der nachzuweisenden Substanzen. Abschnittsmerkmale und Verknüpfungskriterien können substanzspezifisch spezifiziert, und in vorteilhafter Weise adaptiv - zum Beispiel unter Berücksichtigung von Spektrumsmerkmalen des Millieus - optimiert werden. Durch dieses Vorgehen können die nachzuweisenden Substanzen in z.B Anbetracht ihrer charakteristischen Verteilung der Spektrenmaxima mit hoher Zuverlässigkeit unterschieden werden.

Die erfassten Fluoreszenzdaten werden vorzugsweise im Wege einer digitalen Verarbeitung ausgewertet, indem diese mit Rferenzdaten verglichen werden.

Aus dem Datenvergleich, insbesondere aus Korrelationseigenschaften ergibt sich die Identifizierung eines Stoffes.

Die Quantifizierung eines identifizierten Stoffes ergibt sich aus der optischen Dichte des bestrahlten Mediums. In Abhängigkeit von der Konzentration eines im bestrahlten Mediums enthaltenen Stoffes ergeben sich unterschiedliche optische Dichten.

Für veschiedene Stoffe ergeben sich spezifische Signale, Diese Signale werden in Form von Fluoreszenzspektren ermittelt. Die Fluoreszenzspektren können den zu erfassenden Stoffen zugeordnet werden.

Weitere Einzelheiten der Erfindung ergeben sich aus der nachfolgenden Beschreibung in Verbindung mit der Zeichnung. Es zeigen:
- **Fig. 1**: ein hinsichtlich der Intensitätsverteilung des aus vitalem Gewebe remittierten Lichtes charakteristischen Spektrums in einem Wellenlängenbereich von 240 bis 390nm für Amphetamine;
- **Fig.2**: ein hinsichtlich der Intensitätsverteilung des aus vitalem Gewebe remittierten Lichtes charakteristischen Spektrums in einem Wellenlängenbereich von 240 bis 410nm für Benzodiazepine;
- **Fig. 3**: die Intensitätsverteilung des aus Gewebe remittierten Lichtes in einem Wellenlängenbereich von 240 bis 350nm für DL-Methadon (500 ng/ml);
- **Fig. 4**: ein hinsichtlich der Intensitätsverteilung des aus vitalem Gewebe remittierten Lichtes charakteristischen Spektrums in einem Wellenlängenbereich von 250 bis 390nm für Heroin;
- **Fig. 5**: ein hinsichtlich der Intensitätsverteilung des aus vitalem Gewebe remittierten Lichtes charakteristischen Spektrums in einem Wellenlängenbereich von 240 bis 400nm für Kokain;
- **Fig. 6**: ein hinsichtlich der Intensitätsverteilung des aus vitalem Gewebe remittierten Lichtes charakteristischen Spektrums in einem Wellenlängenbereich von 250 bis 390nm für LSD und Nor-LSD;
- **Fig. 7**: ein hinsichtlich der Intensitätsverteilung des aus vitalem Gewebe remittierten Lichtes charakteristischen Spektrums in einem Wellenlängenbereich von 250 bis 390nm für Methadon D3;
- **Fig. 8**: ein hinsichtlich der Intensitätsverteilung des aus vitalem Gewebe remittierten Lichtes charakteristischen Spektrums in einem Wellenlängenbereich von 250 bis 500nm für ausgewählte Opiate;
- **Fig. 9**: ein hinsichtlich der Intensitätsverteilung des aus vitalem Gewebe remittierten Lichtes charakteristischen Spektrums in einem Wellenlängenbereich von 240 bis 400nm für Strassenheroin mit Kokain;
- **Fig. 10**: ein hinsichtlich der Intensitätsverteilung des aus vitalem Gewebe remittierten Lichtes charakteristischen Spektrums in einem Wellenlängenbereich von 250 bis 390nm für delta9-THC;
- **Fig. 11**: ein hinsichtlich der Intensitätsverteilung des aus vitalem Gewebe remittierten Lichtes charakteristischen Spektrums in einem Wellenlängenbereich von 220 bis 310nm für Dextromethorphan;
- **Fig. 12**: ein hinsichtlich der Intensitätsverteilung des aus vitalem Gewebe remittierten Lichtes charakteristischen Spektrums in einem Wellenlängenbereich von 220 bis 450nm für die Benzodiazepinanaloga Zolpidem und Zopiclon, das Neuroleptikum Promethazin und das trizyklische Antidepressivum Doxepin;
- **Fig. 13**: ein hinsichtlich der Intensitätsverteilung des aus vitalem Gewebe remittierten Lichtes charakteristischen Spektrums in einem Wellenlängenbereich von 200 bis 440nm für Oxcarbazepin;
- **Fig. 14**: ein hinsichtlich der Intensitätsverteilung des aus vitalem Gewebe remittierten Lichtes charakteristischen Spektrums in einem Wellenlängenbereich von 400 bis 870nm für Gabapentin;
- **Fig. 15**: ein hinsichtlich der Intehsitätsverteilung des aus vitalem Gewebe remittierten Lichtes charakteristischen Spektrums in einem Wellenlängenbereich von 200 bis 400nm für Carbamazepin;
- **Fig. 16**: ein hinsichtlich der Intensitätsverteilung des aus vitalem Gewebe remittierten Lichtes charakteristischen Spektrums in einem Wellenlängenbereich von 200 bis 350nm für Buprenorphin;
- **Fig. 17**: Mittelwertspektren von menschlichem Blut für einen Wellenlängenbereich von 240 bis 480nm für Cannabinoide, Kokain mit dessen Abbauprodukten (Ecgonine) und Amphetamine;
- **Fig. 18**: die gewichteten, nicht-linearen zweiten Ableitungen von Cannabinoiden, Kokain mit dessen Abbauprodukten (Ecgonine) und Amphetaminen in einem Wellenlängenbereich von 220 bis 420nm;
- **Fig. 19-21**: die paarweisen Gradienten der gewichteten, nicht-linearen Ableitungen z.B. für die Gruppe des Kokains und der Ecgonine, die Gruppe der Amphetamine und die Gruppe der Cannabinoide in einem Wellenlängenbereich von 220 bis 420nm;
- **Fig. 22**: ein hinsichtlich der Intensitätsverteilung des aus vitalem Gewebe remittierten Lichtes charakteristischen Spektrums in einem Wellenlängenbereich von 200 bis 550nm für DL-Methadon, L-Methadon und D-Methadon;
- **Fig. 23**: zeigt für Kokain das Fluoreszenzspektrum von, aus vitalem Gewebe remittierten Licht in einem im Wellenlängenbereich von 240 bis 740 nm;
- **Fig. 24**: zeigt für LSD das Fluoreszenzspektrum von, aus vitalem Gewebe remittierten Licht in einem Wellenlängenbereich von 300 bis 630nm;
- **Fig. 25**: zeigt für 11 Hydroxy-THC das Fluoreszenzspektrum von, aus vitalem Gewebe remittierten Licht in einem Wellenlängenbereich von 240 bis 880nm;
- **Fig. 26**: zeigt das Fluoreszenz-Spektrum von Heroin in einem aus vitalem Gewebe remittierten Licht in einem Wellenlängenbereich von 300 bis 825nm;

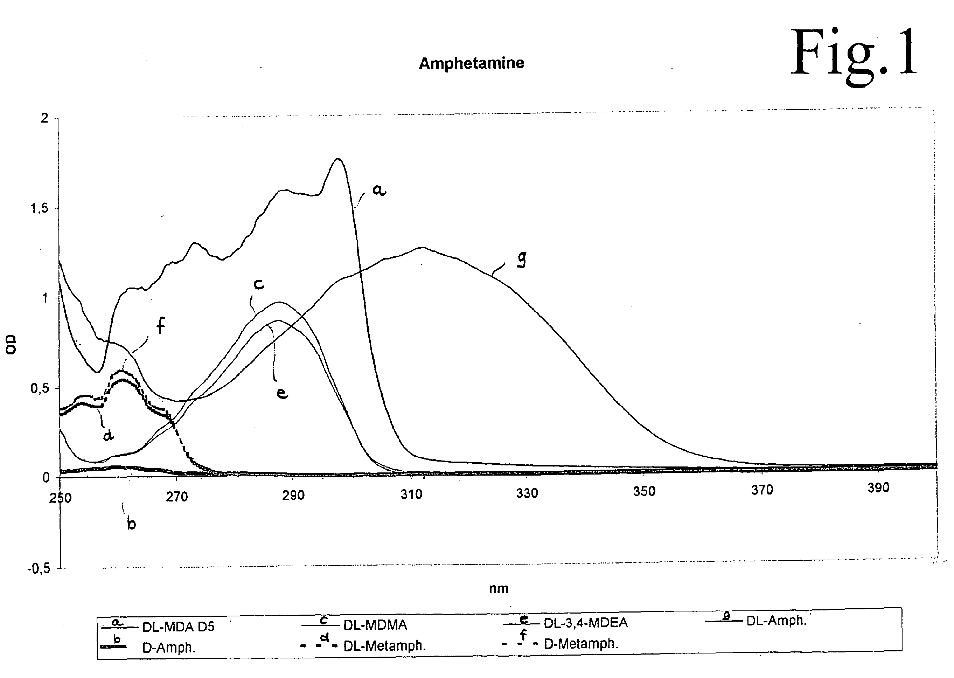

Fig. 1 zeigt ein hinsichtlich der Intensitätsverteilung des aus vitalem Gewebe remittierten, und durch eine Messeinrichtung erfassten Lichtes, für unterschiedliche Amphetamine.

Der Graph a zeigt das Remissionslichtspektrum für DL-MDA D5. Der Graph a weist bei einer Wellenlänge von 256nm ein charakteristisches lokales Minimum auf. Bei Wellenlängen von 275nm, 250nm und 296nm ergeben sich markante lokale Maxima der optischen Dichte des erfassten Remissionslichtes. Die optische Dichte des erfassten Remissionslichtes liegt für die Wellenlänge von 256nm bei 0,6, für die Wellenlänge 274nm bei 1,25, für die Wellenlänge von 286nm bei 1,5 und für die Wellenlänge von 298nm bei 1,7.

Graph b beschreibt das Remissionslichtspektrum für D-Amphetamin in einer Konzentration von 500 ng/ml. Der für das D-Amphetamin charakteristische Graph b, weist bei einer Wellenlänge von 260nm ein lokales Maximum auf. Im Vergleich zu den weiteren beschriebenen Amphetaminen ist das D-Amphetamin selbst bei der hier gezeigten Konzentration von 500 ng/ml in dem Wellenlängenfenster von 250 bis 400nm in soweit zuverlässig nachweisbar, als das sich das für D-Amphetamin charakteristische Spektrum deutlich von den ansonsten für Amphetamine typischen Spektren unterscheidet und insbesondere bei 260nm ein Maximum aufweist.

Der Graph c zeigt ein charakteristisches Remissionslichtspektrum für DL-MDMA in einer Konzentration von 500 ng/ml. Der Graph c hat bei einer Wellenlänge von 286nm ein charakteristisches absolutes Maximum. Der Graph c weist im Bereich von 260 bis 310nm keine weiteren lokalen Extremwerte auf. Der Graph c zeichnet sich durch einen zum absoluten Maximum bei 286nm im wesentlichen symmetrischen, glockenartigen Verlauf aus. Das absolute Maximum des DL-MDMA liegt im Bereich einer zentral nervös relevanten Konzentration in etwa im Bereich des lokalen Maximum des DL-MDA (Graph a).

Der Graph d zeigt einen für DL-Metamphetamin charakteristischen Verlauf der optischen Dichte über der Wellenlänge. Bei einer Wellenlänge von 257nm ergibt sich ein charakteristisches lokales Minimum und bei einer Wellenlänge von 261nm ein charakteristisches absolutes Maximum. Im Wellenlängenbereich von 265nm sowie bei 269nm ergeben sich für die zweite Ableitung der optischen Dichte nach der Wellenlänge charakteristische Maxima.

Der Graph e verdeutlicht ein für DL-3,4-MDEA charakteristisches Remissionslichtspektrum bei einer Konzentration von 500 ng/ml. Der Graph e zeichnet sich aus durch ein lokales Minima bei einer Wellenlänge im Bereich von 255nm. Bei einer Wellenlänge im Bereich von 286nm ergibt sich ähnlich wie bei DL-MDMA (500 ng/ml) ein lokales Maximum. Jenseits einer Wellenlänge von 310nm liegt die optische Dichte des Remissionslichtes für DL-3,4-MDEA (500 ng/ml) nahezu bei 0.

Der Graph f beschreibt den Verlauf des Remissionslichtspektrums für D-Metamphetamin (500ng/ml). Bei einer Wellenlänge von 254nm ergibt sich ein lokales Maximum. Bei einer Wellenlänge von 257nm ergibt sich ein lokales Minimum. Bei einer Wellenlänge von 260nm ergibt sich ein absolutes Maximum. Bei Wellenlängen von 265nm sowie 269nm ergeben sich hinsichtlich der Ableitung der optischen Dichte über der Wellenlänge Maxima.

Graph g zeigt ein für ein DL-Amphetamin bei einer Konzentration von 500 ng/ml signifikantes Remissionslichtspektrum. Signifikant ist ein bei einer Wellenlänge von 270nm liegendes lokales Minima, sowie ein bei einer Wellenlänge von 262nm liegendes Minimum und ein bei einer Wellenlänge von 312nm liegendes Maximum.

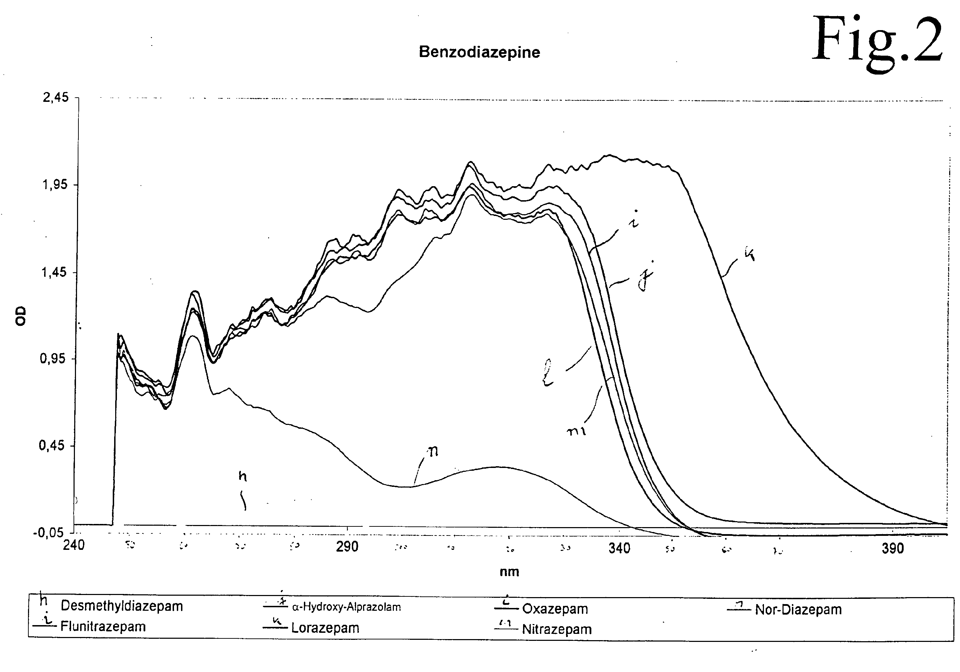

Fig.2 zeigt das Remissionslichtspektrum für mehrere, zur Gruppe der Benzodiazepine gehörende psychoaktiven Substanzen. Graph h beschreibt das Remissionslichtspektrum für Desmethyldiazepam in einer Konzentration von 500 ng/ml. Charakteristisch für diese Substanz ist insbesondere das Spektrum im Bereich von 240 bis 300nm. So ergibt sich bei einer Wellenlänge von 254nm ein lokales Minimum und bei einer Wellenlänge von 282nm ein absolutes Maximum. Jenseits einer Wellenlänge von 294nm zeigt diese Substanz keinen Spektralanteil.

Der Graph i beschreibt den Verlauf eines für Flunitrazepam (500ng/ml) charakteristischen Verlauf des Remissionslichtspektrums.

Der Graph j zeigt den Verlauf des Remissionslichtspektrums für α-Hydroxy-Alprazolam.

Der Graph k zeigt das Remissionslichtspektrum für Lorazepam in einer Konzentration von 500 ng/ml.

Der Graph I zeigt den Verlauf für Oxazepam in einer Konzentration von (500 ng/ml).

Der Graph m zeigt den Verlauf des Remissionslichtspektrums für Nitrazepam (500 ng/ml).

Der Graph n zeigt den Verlauf des Remissionslichtspektrums in einem Wellenlängenbereich von 240 bis 400nm für Nor-Diazepam ebenfalls in einer Konzentration von 500 ng/ml.

Hinsichtlich der für die einzelnen Remissionlichtspektren charakteristischen Merkmale wird ausdrücklich auf die Fig. 2 verwiesen. Die Erkennung der in vitalem Gewebe befindlichen psychoaktiven Substanzen erfolgt vorzugsweise durch kombinierte Anwendung mehrerer Korrelationskriterien.

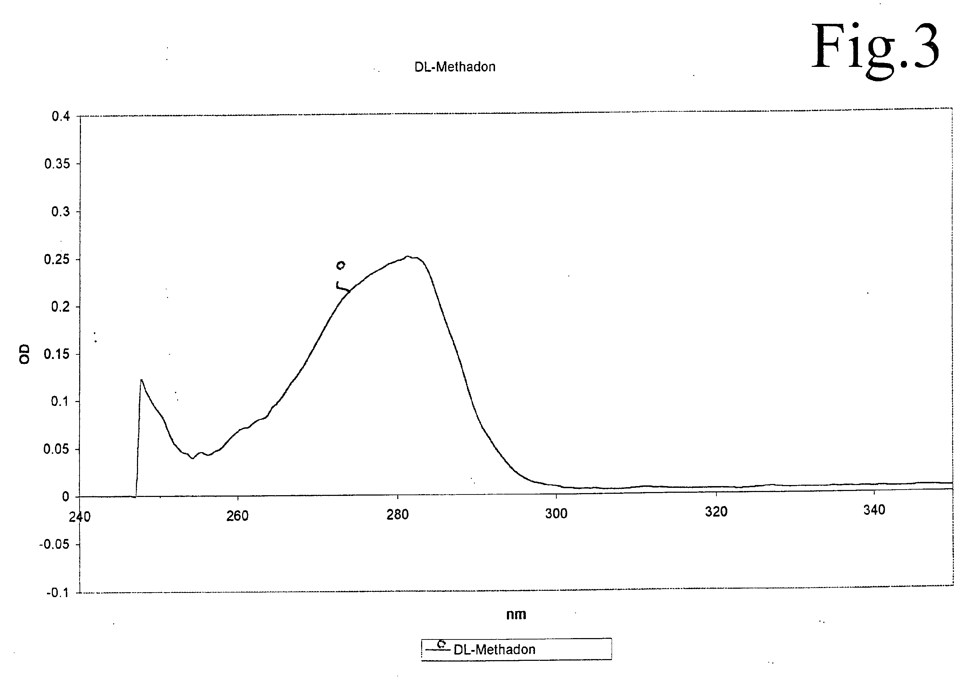

Fig. 3 zeigt das Remissionlichtspektrum für DL-Methadon in einer Konzentration von (500 ng/ml). Ein charakteristisches lokales Minimum ergibt sich hierbei bei einer Wellenlänge von 254nm. Bei einer Wellenlänge im Bereich von 282nm ergibt sich ein absolutes Maximums mit einer optischen Dichte von 0,26. Im Umgebungsbereich dieses absoluten Maximums ergibt sich ein glockenartiger Verlauf des Graphen.

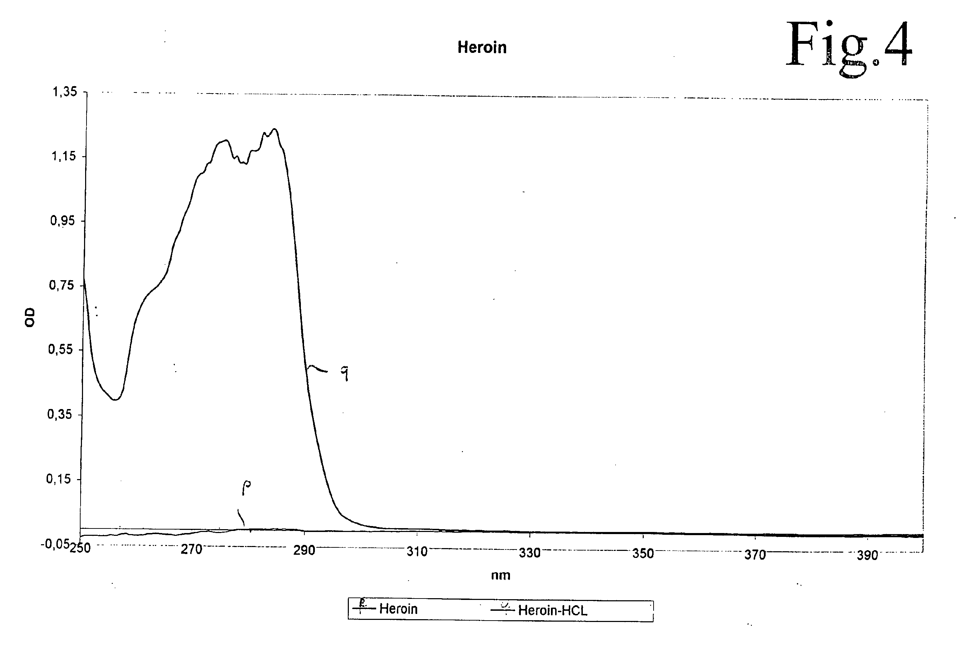

Fig. 4 zeigt das Remissionslichtspektrum für Heroin und zwar für Heroin in einer Konzentration von 500 ng/ml (Graph p) sowie Heroin-HCL in einer Konzentration von 50 ng/ml (Graph q).

Für das Heroin-HCL ergibt sich bei 256nm ein charakteristisches lokales Minimum. Bei 275nm sowie bei 285nm ergeben sich charakteristische lokale Maxima. Zwischen diesen beiden lokalen Maxima liegt auf hohem Niveau ein lokales Minimum mit einer Wellenlänge von 279nm. Weitere, für Heroin-HCL signifikante Merkmale des Remissionslichtspektrums, sind unmittelbar aus Fig. 4 ersichtlich (siehe hierbei insbesondere die im Bereich von 290 bis 305nm liegende stark abfallende Flanke).

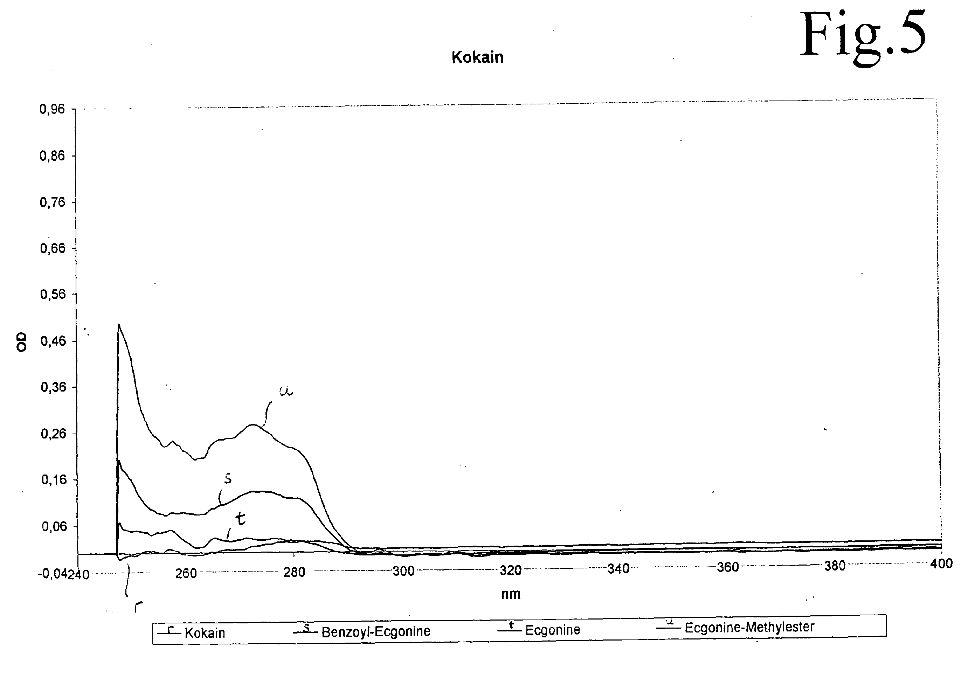

Fig.5 offenbart ein Remissionlichtspektrum zur Identifizierung von Kokain und seinen charakteristischen psychoaktiven Metaboliten. Der Graph r zeigt hierbei den Verlauf von Kokain in einer Konzentration von 100 ng/ml. Das Spektrum ist durch ein geringes Maximum bei 284nm gekennzeichnet. Graph s zeigt den Verlauf von Benzoyl-Ecgonine 100 ng/ml, Graph t zeigt das Remissionslichtspektrum für Ecgonine 100 ng/ml. Der Graph u zeigt den Verlauf des Remissionlichtspektrums für Ecgonine-Methylester in einer Konzentration von 100 ng/ml.

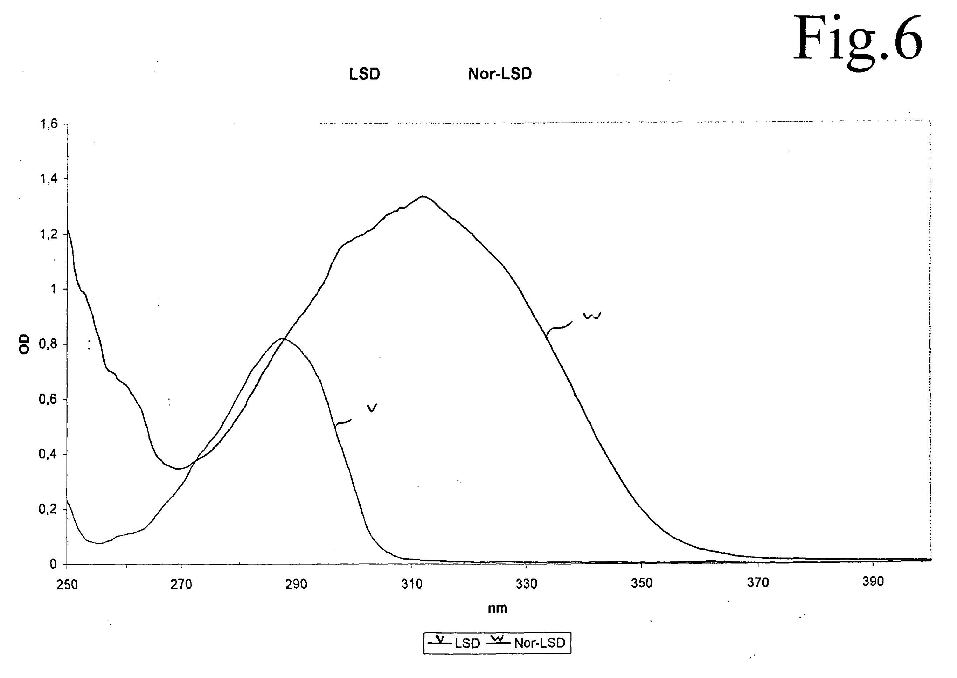

Fig. 6 zeigt ein für den Nachweis von LSD geeignetes Spektralfenster in einem Wellenlängenbereich von 250 bis 400nm. Für LSD ergibt sich hierbei bei einer Wellenlänge von 256nm ein lokales Minimum und bei einer Wellenlänge von 288nm ein absolutes Maximum. Im Umgebungsbereich dieses absoluten Maximums ergibt sich ein im wesentlichen glockenförmig abfallender Verlauf.

Für Nor-LSD (Graph w) ergibt sich bei 269nm ein lokales Minimum und bei 310nm ein absolutes Maximum. Weitere, für den Nachweis der Stoffe LSD und Nor-LSD geeignete Kurveneigenschaften sind aus der Darstellung nach Figur 6 ersichtlich. Die Differenz der Kurvenmaxima zwischen LSD und seinem Abbauprodukt Nor-LSD beträgt damit 31nm, so daß ein Nachweis der Muttersubstanz und des Abbauproduktes möglich wird.

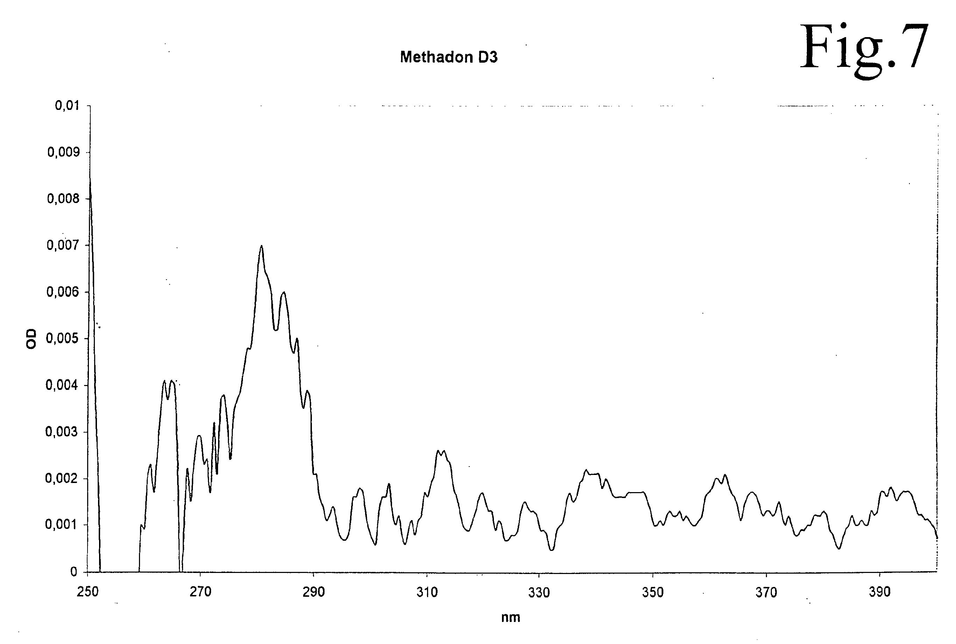

In Fig. 7 zeigt in hoher Auflösung ein Remissionslichtspektrum von Methadon D3. Die optische Dichte erreicht maximal 0,007 bei 279nm. Die Lichtintensität erweist sich damit als sehr gering, die Identifizierung dieser Substanz im vitalen Gewebe ist insbesondere im Hinblick auf die Charakteristik im Wellenlängenbereich von 286 bis 320 mit ausreichender Zuverlässigkeit möglich. Dieses Remissionslichtspektrum weist insbesondere bei den Wellenlängen von 279nm, 312nm, 340nm und 362nm charakteristische Merkmale auf, durch welche die Identifizierung des Methadon D3 weiterhin ermöglicht wird.

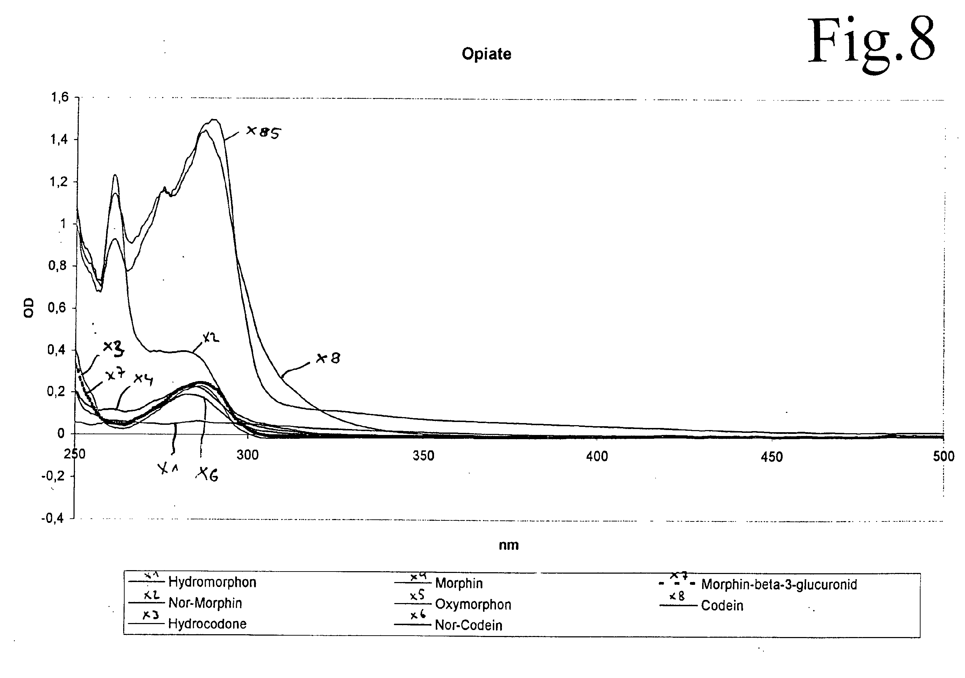

In Fig. 8 sind mehrere zur erfindungsgemäßen Identifikation von Opiaten signifikante Spektren dargestellt. Der Graph x1 betrifft Hydromorphon in einer Konzentration von (500 ng/ml). Der Graph x2 betrifft Nor-Morphin ebenfalls in einer Konzentration von 500 ng/ml. Der Graph x3 betrifft Hydrocodone bei einer Konzentration von 500 ng/ml. Der Graph x4 zeigt Morphin in einer Konzentration von 50 ng/ml. Der Graph x5 zeigt Oxymorphon in einer Konzentration von 500 ng/ml. Der Graph x6 zeigt Nor-Codein in einer Konzentration von 500 ng/ml. Der Graph x7 zeigt Morphin-beta-3-glucoronid in einer Konzentration von 50 ng/ml. Der Graph x8 zeigt Codein in einer Konzentration von 500 ng/ml.

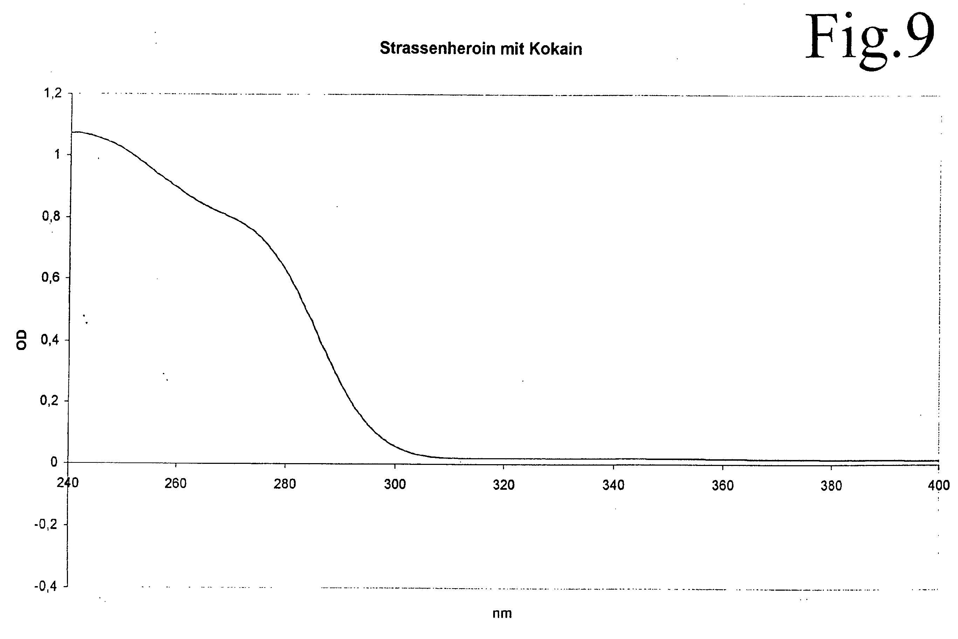

In Fig. 9 zeigt den Verlauf des Remissionlichtspektrums für Straßenheroin mit Kokain. Für diese psychoaktive Substanz liegt ein ausreichend indikativer Bereich in einem Wellenlängenabschnitt von 240 bis 310nm vor. Bei 270nm liegt das Maximum eines überlagerten Remissionsspektrums von Kokain. In der Überlagerung sind insbesondere Streckmittel enthalten.

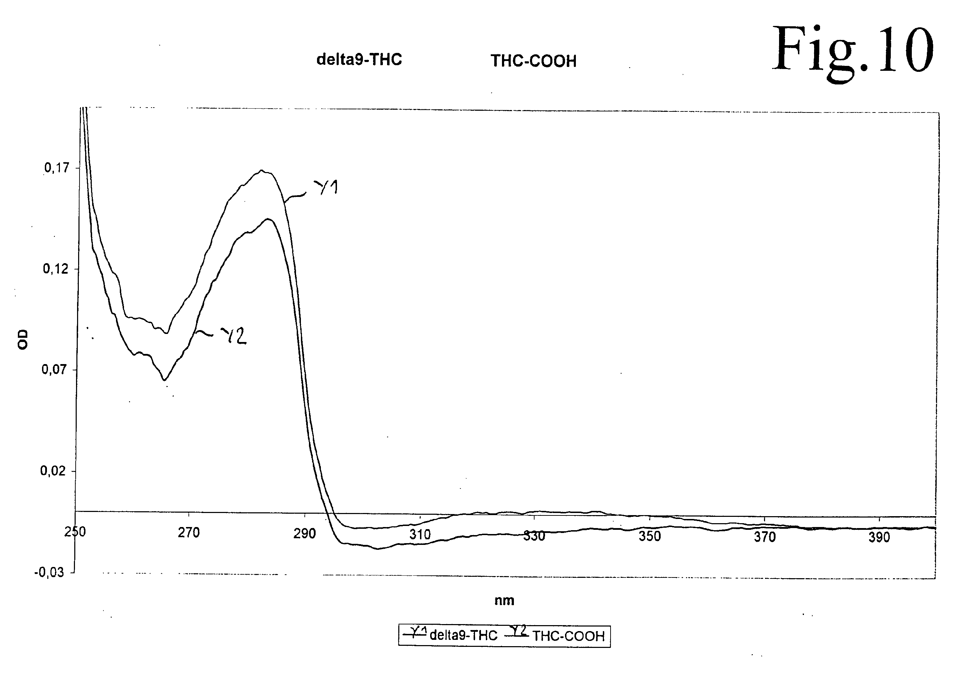

In Fig. 10 ist das Remissionslichtspektrum für delta 9-THC (Graph y1) und THC-COOH (Graph y2) in einer Konzentration von 50 ng/ml dargestellt. Die Maxima der Spektren für beide Substanzen liegen bei 283nm (y2) und 280nm (y1). Für die Unterscheidung der Substanzen ist die Verschiebung der Spektren hinsichtlich der Anstiegsphase im Bereich von 270 bis 280nm und der Abstiegsphase im Bereich vom 280 bis 295nm charakteristisch.

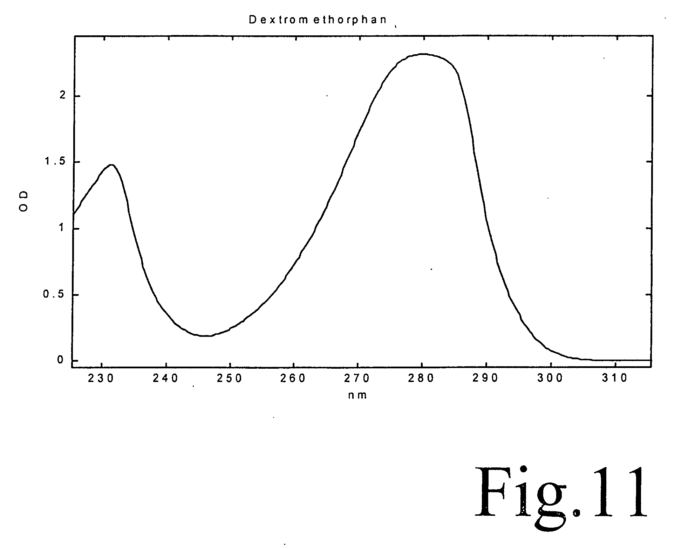

Fig. 11 zeigt den Verlauf des Remissionsspektrums für die psychoaktive Substanz Dextromethorphan in einer Konzentration von 100 ng/ml. Charakteristisch für diese Substanz ist insbesondere das breite lokale Minimum mit einer optischen Dichte bei einer Wellenlänge von 246nm und das absolute Maximum der optischen Dichte bei einer Wellenlänge von 280nm sowie das lokale Maximum der optischen Dichte bei einer Wellenlänge von 232nm.

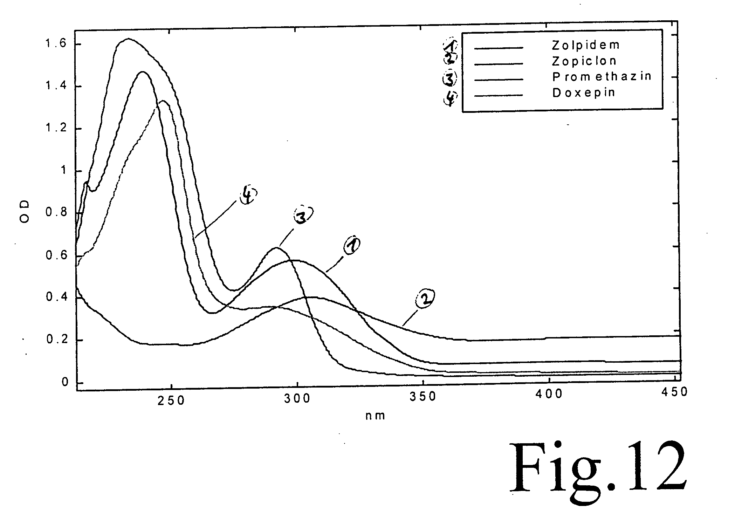

Fig.12 zeigt die zur erfindungsgemäßen Erfassung der psychoaktiven Substanzen Zolpidem, Zopiclon, Promethazin und Doxepin charakteristischen Remissions-Lichtspektren. Auch für diese Substanzen ergibt sich insbesondere im Wellenlängenbereich von 200 bis 360nm eine besonders hohe Aussagefähigkeit. Die Substanzen sind eindeutig unterscheidbar. Die optische Dichte im definierten Wellenlängenbereich ist ausreichend, um eine zuverlässige quantitative Bestimmung der Substanzen zu ermöglichen. Die Benzodiazepinanaloga Zolpidem und Zopiclon zeigen ähnliche spektroskopische Eigenschaften wie Benzodiazepine aufgrund ihrer ähnlichen chemischen Eigenschaften.

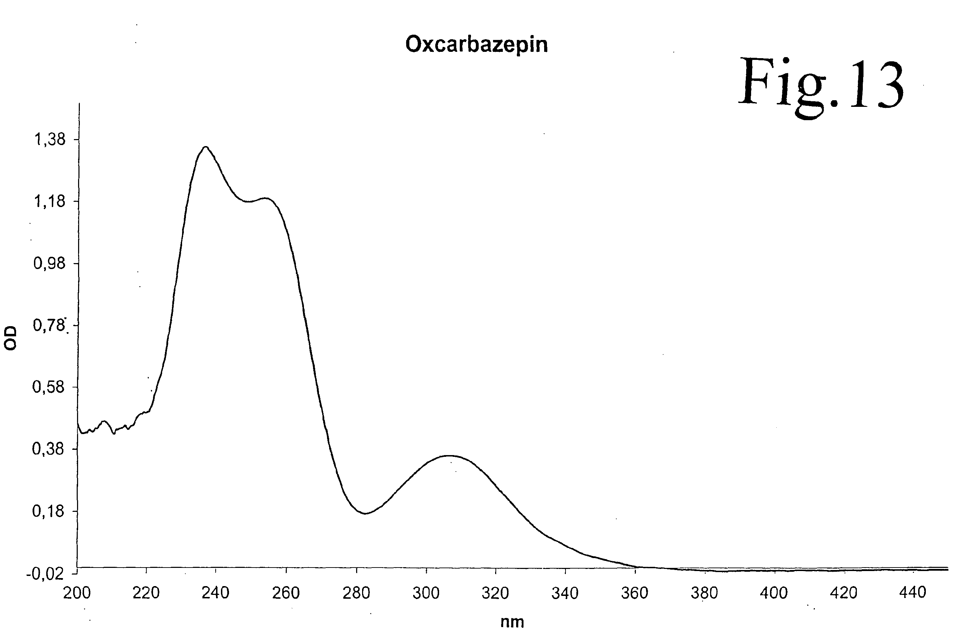

Fig.13 zeigt das Spektrum von Oxcarbazepin. Charakteristisch sind ein Maximum bei 238nm, ein Maximum bei 255nm, ein Minimum bei 281nm und ein weiteres Maximum bei 308nm.

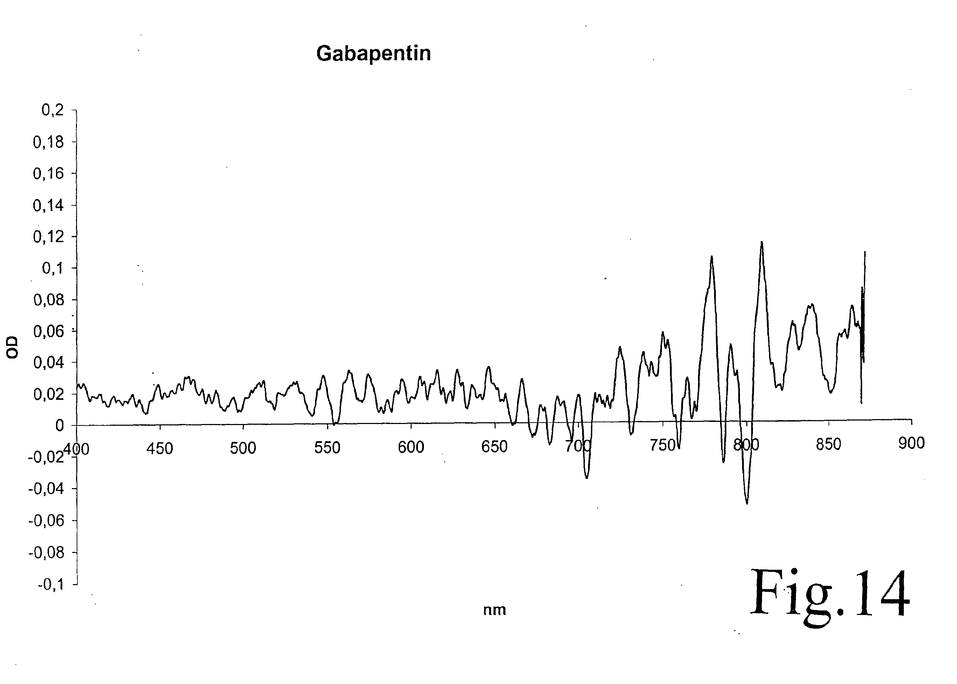

Fig.14 zeigt das Spektrum für Gabapentin im Wellenlängenbereich von 400 bis 865nm. Die hohe Welligkeit des Graphen in diesem speziellen Verlauf ist signifikant für diese Substanz. Es handelt sich nicht um Artefakte wie z.B. Rauschen. Die für die Substanz charakteristischen Maxima und Minima oder vorzugsweise der gesamte Verlauf des Spektrums wird vorzugsweise in hoher Auflösung digitalisiert gespeichert und als Referenzdatensatz bereitgehalten.

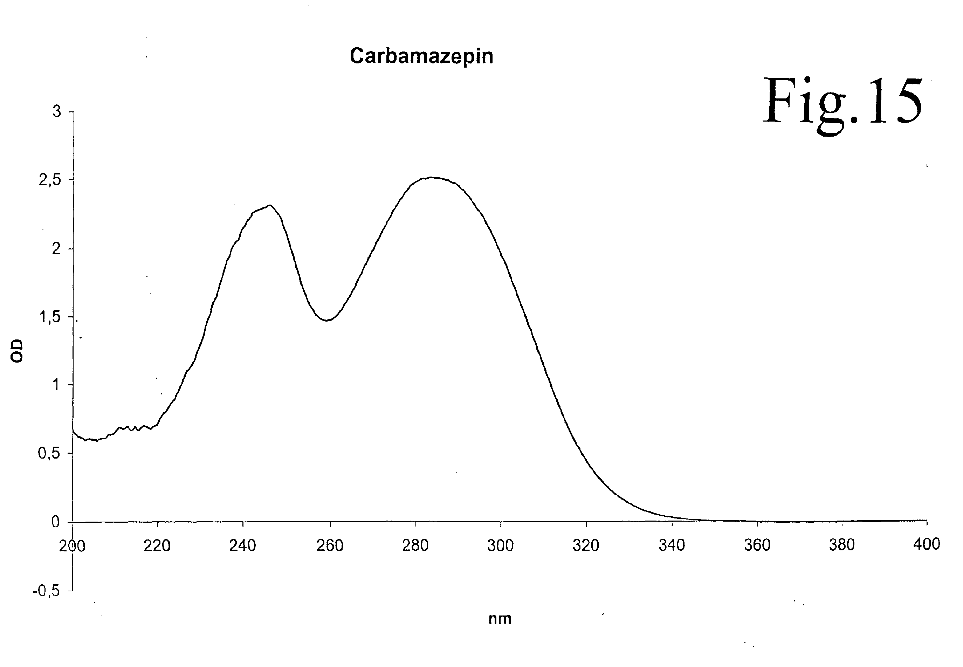

Fig.15 zeigt das für Carbamazepin charakteristische Spektrum mit einem zweigipfligen Verlauf. Das erste Maximum liegt bei 245nm, das zweite Maximum bei 284nm. Ein spezifisches Minimum tritt bei 249nm auf.

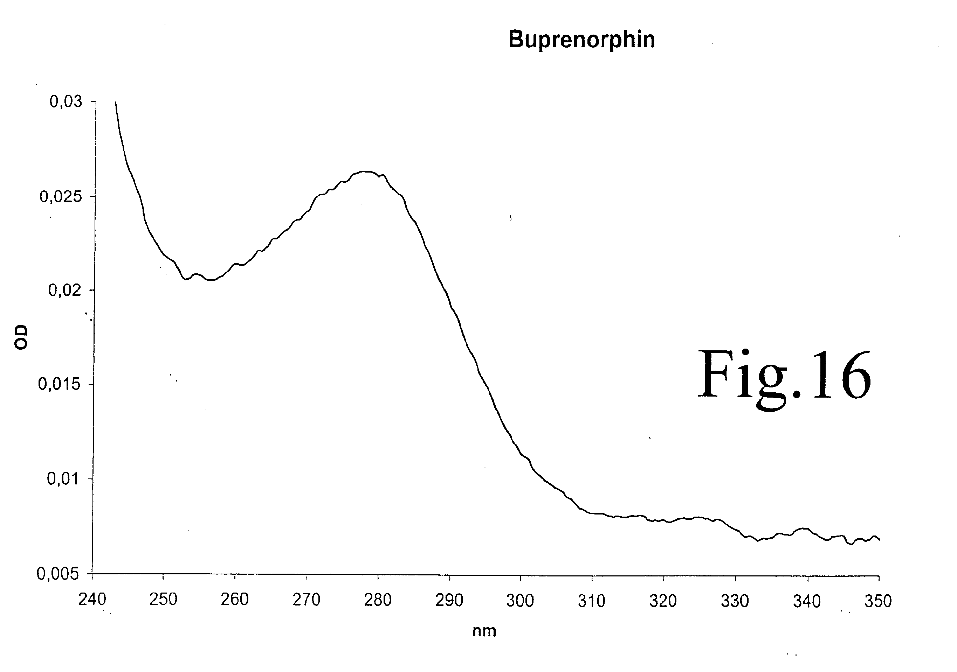

Fig.16 zeigt das für Buprenorphin charakteristische Spektrum in einem Wellenlängenbereich von 240 bis 350nm. Es sind feinwellige, das Spektrum überlagernde Feinstrukturspektren ersichtlich. Das charakteristische Maximum bei 277nm und bei 224nm entsteht durch die Photoaktivität der funktinellen Gruppe im Molekül der Substanz.

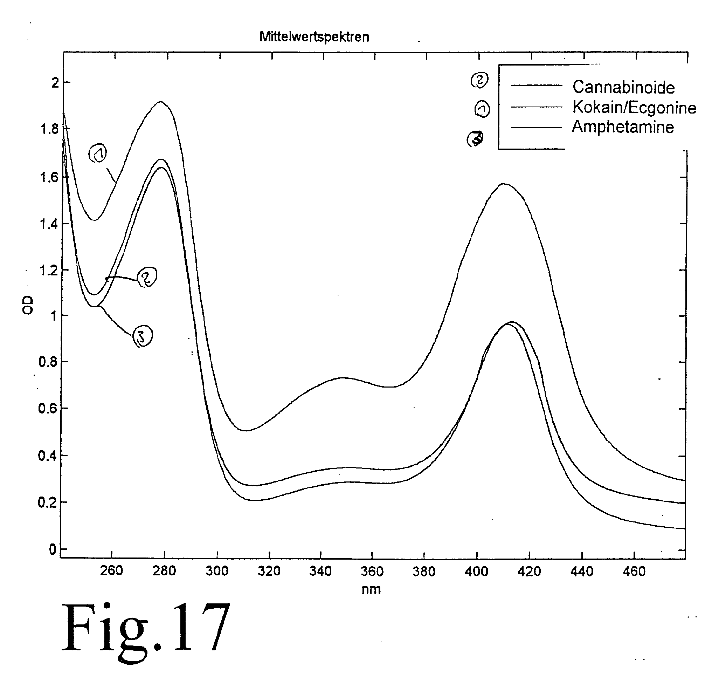

Fig.17 zeigt die Mittelwertspektren der drei verschiedenen Substanzgruppen Cannabinoide, Kokain/Ecgonine und Amphetamine von Konsumenten einer jeweiligen Substanzgruppe. Bei etwa 280nm absorbieren die Serumproteine maximal und bei ca. 420nm das Hämoglobin. Feinstrukturen sind in dieser Darstellung unterdrückt.

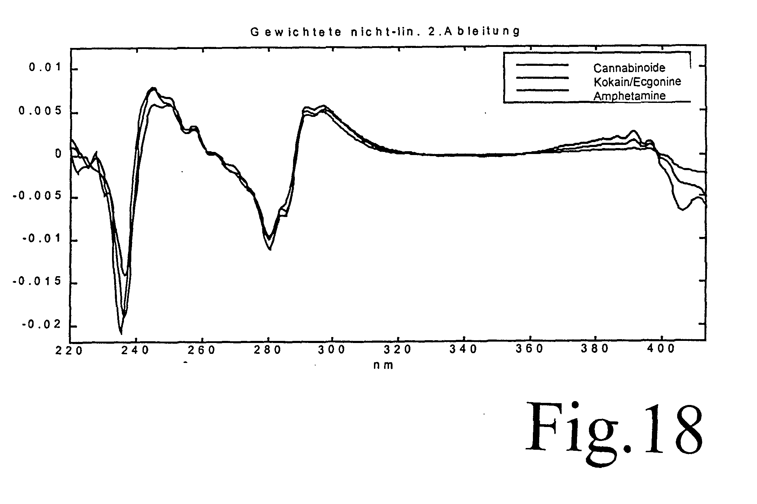

Bei Figur 18 handelt es sich um eine Darstellung der gewichteten, nicht - linearen 2. Ableitungen der Ausgangsspektren aus Figur 17. Bei 280nm sind deutliche Minima zu sehen. Diese werden durch Serumproteine erzeugt. Die Maxima im Kurvenverlauf liegen bei 238 nm.

Es ergeben sich insbesondere dann die Detektierbarkeit und eindeutige Zuordnungen eines Spektrums zu einer Substanz oder einer Substanzgruppe, wenn paarweise Gradienten der gewichteten, nicht-linearen Ableitungen z.B. für die Gruppe des Kokains und der Ecgonine, die Gruppe der Amphetamine und die Gruppe der Cannabinoide gebildet werden. Wenn diese Gradientenbildung ergibt, dass für jeweils eine Substanz oder eine chemisch verwandte Substanzgruppe Absorbtionsmaxima bei bestimmten, für diese Substanz oder Substanzgruppe charakteristischen Wellenlängen nachweisbar sind, dann sind diese Maxima Resultat gleicher Substanzen oder Substanzgruppen.

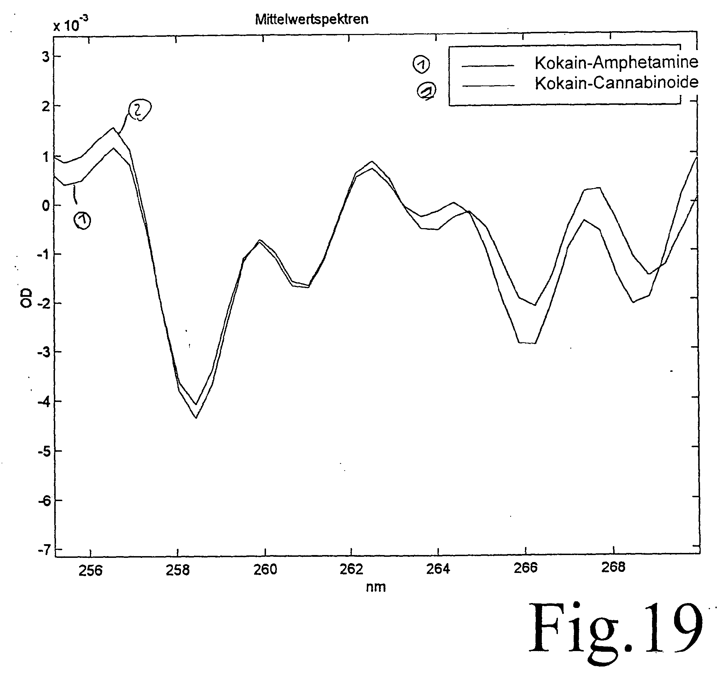

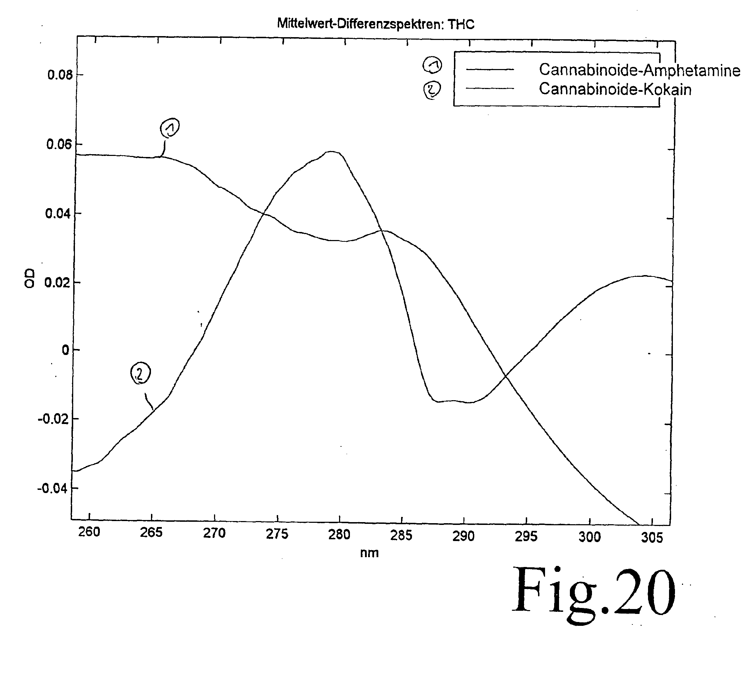

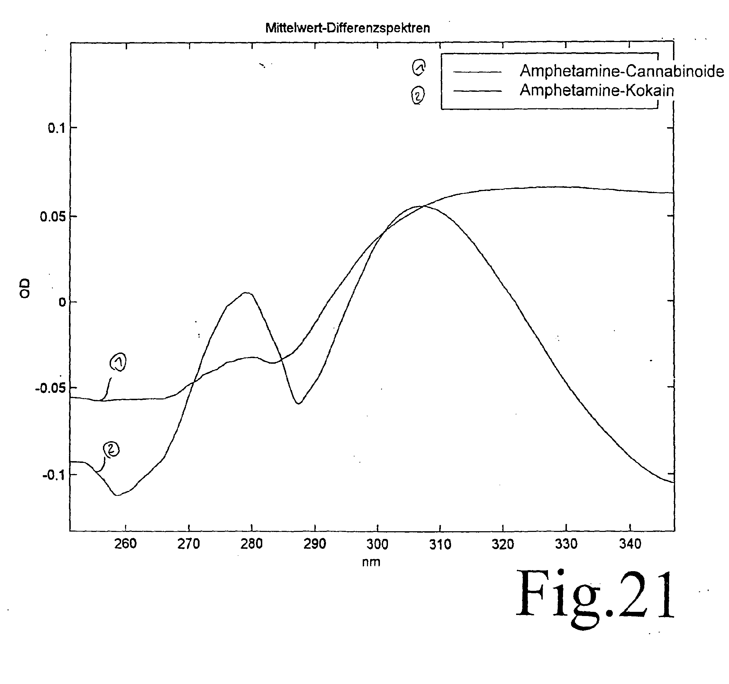

Dieser Zusammenhang ist für Kokain und Ecgonine in der Figur 19, für Cannabinoide in Figur 20 und für Amphetamine in Figur 21 dargestellt.

Durch die paarweise Gradientenbildung von Kokain und Ecgoninen mit Amphetaminen beziehungsweise durch die paarweise Gradientenbildung von Kokain und Ecgoninen mit Cannabinoiden ergeben sich, wie Figur 19 zeigt, Absorbtionsmaxima bei 259nm, bei 266nm und 268,5nm.

Figur 20 zeigt das Resultat der paarweisen Gradientenbildung für die Cannabinoide. Maximale Absorptionen zeigen sich bei 279nm und bei 284nm. Das Maximum bei 305nm ist Resultat des zusätzlichen Vorkommens eines Benzodiazepines.

Aus Figur 21 geht hervor, dass sich durch paarweise Gradientenbildung der nicht-linearen 2. Ableitungen für die Gruppe der Amphetamine spezifische Maxima bei 278,5nm und im Bereich von 307nm ergeben. Die vorhandenen Konzentrationen erlauben eine eindeutige Quantifizierung der Substanzgruppen.

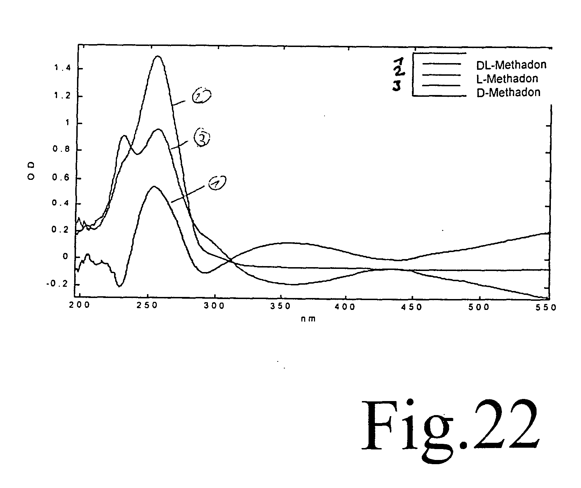

Fig.22 veranschaulicht die reflexionsspektroskopischen Eigenschaften des racemischen Gemisches bestehend aus gleichen Anteilen des reinen L-Methadonenantiomeres und des reinen D-Methadonenantiomeres. L- Methadon weist ein eingipfeliges Maximum im Wellenlängenbereich von 256nm auf. D-Methadon und DL-Methadon zeigen zweigipfelige Lichtspektren: D-Methadon bei 238nm und bei 256nm, DL-Methadon bei 254,5nm und bei 350nm. Das D-Enantiomer zeigt zusätzlich ein überlagertes charakteristisches Spektrum im Wellenlängenbereich von 302nm.

Durch die erfindungsgemäße Generierung eines Remissionlichtspektrums wird es möglich, psychoaktive Substanzen in äußerst geringen Stoffkonzentrationen zuverlässig zu detektieren. Es ist möglich, die sich zu einem Gesamtremissionslichtspektrum überlagernden Einzelspektren eindeutig zu trennen. Die Auswertungsergebnisse stehen nach kürzester Auswertungszeit zur Verfügung. Die Aufzeichnung des Remissionlichtspektrums kann in vorteilhafter Art auf nicht-invasive Weise erfolgen. Damit ergibt sich ein kostengünstiges und besonders aussagefähiges Screening-Verfahren zum Psychopharmaka (Neuroleptika, Antidepressiva, Sedativa und Hypnotika) Antiepileptika und Antibiotika sowie deren Metabolite.

Durch das erfindungsgemäße Selektionsverfahren wird die Möglichkeit geschaffen, das Vorhandensein von illegalen Drogen und Arzneimitteln der genannten Arzneimittelgruppen im menschlichen Organismus ohne Blutabnahme, Haaranalyse oder Urinuntersuchungen nachzuweisen. Aus den im Zusammenhang mit der Erprobung der erfindungsgemäßen Lösung durchgeführten Untersuchungen ergibt sich, dass das erfindungsgemäße Verfahren sich zur Routineerfassung illegaler Substanzen im Blut eignet. Das durch eine vergleichsweise kostengünstig aufgebaute Hardware durchführbare Analyseverfahren eignet sich damit für Routinekontrollen im Straßenverkehr, zum Nachweis von illegalen Drogen und anderen, die Fahrtauglichkeit einschränkenden Medikamenten und psychoaktiven Substanzen.

Da durch das erfindungsgemäße Verfahren, sowohl die Substanzen an sich als auch deren Konzentration auf zuverlässige Weise ermittelt werden können, wird eine qualitative Unterscheidbarkeit auch innerhalb von Substanzgruppen ermöglicht. Insbesondere ist es auch möglich, Metabolite der psychoaktiven Substanzen zuverlässig zu erfassen. Das erfindungsgemäße Verfahren eignet sich in dieser Hinsicht insbesondere zur Detektion von Cannabinoiden, Amphetaminen und Kokain. Insbesondere in einer für die Straßenverkehrstauglichkeit eines Menschen relevanten Konzentration, können die psychoaktiven Substanzen routinemäßig durch Belichtung der Haut der zu untersuchenden Person nachgewiesen werden. Weitere Anwendungsgebiete sind die Spiegelkontrolle von Antiepileptika, welche spiegelkontrolliert beim Menschen eingesetzt werden. Drugmonitoring gestützte pharmakotherapeutische Interventionsmöglichkeiten sind auf dem Gebiet der Antibiotikatherapie und der Drugmonitoring gestützten Psychopharmakotherapie mit Neuroleptika und Antidepressiva. Die sich hieraus ergebenden individuellen Dosierungsmöglichkeiten bilden als solche die Grundlage für eine individuell abgestimmte medikamentöse Therapie. Eine derartige Therapie kann auch für sich gesehen erfindungswesentlich sein. Die Risiken von Über- und Unterdosierungen werden auf diese Weise ausgeschaltet.

Eine besonders zuverlässige Auswertung der in-vivo aufgenommenen Reflexionsspektren erfolgt vorzugsweise durch eine zentrale Auswertungseinheit. Die hinsichtlich des aufgenommenen Remissionlichtspektrums indikativen Datensätze können hierbei über eine mobile Kommunikationseinrichtung an einen zentralen Rechner, gegebenenfalls über das Internet weitergeleitet werden. An Hand des zentralen Rechners können umfangreiche Korrelationalgorithmen abgearbeitet werden, um aus dem Remissionslichtspektrum die Art und die Konzentration etwaiger unzulässiger Substanzen im menschlichen Organismus zu errechnen. Hierbei ist es möglich bekannte Matrix-Effekte ebenfalls zu berücksichtigen.

Auf Grund der durch das erfindungsgemäße Verfahren möglich werdenden Erfassung und Quantifizierung der Substanzgruppen wird es möglich, den momentanen physiologischen Zustand der zu untersuchenden Person zu beurteilen. Es hat sich gezeigt, dass es durch das erfindungsgemäße Verfahren auch möglich wird, Metabolite der psychoaktiven Substanzen transdermal im Kapillarbett zuverlässig zu erfassen. Das erfindungsgemäße Verfahren beruht auf dem Prinzip, dass Licht unterschiedlicher Wellenlängen unterschiedlich tief in das belebte Gewebe eindringt. Insbesondere dringt längerwelliges (energieärmeres) Licht tiefer als kürzerwelliges (energiereicheres) Licht in das Gewebe ein. Dies bedeutet, dass kurzwelliges Licht an der gleichen Meßstelle weniger Substanz erreicht als langwelliges Licht. Innerhalb des Spektrums vom kurzwelligen zum langwelligen Licht, erhält man einen Konzentrationsgradienten. Das erfindungsgemäße Messverfahren zur Bestimmung der Konzentration von psychoaktiven Substanzen, insbesondere Chromophoren im Gewebe nutzt in vorteilhafter Weise diesen Effekt.

Wird nun die gleiche Meßstelle mit Licht unterschiedlicher Intensität beleuchtet, so erhält man Spektren die aus unterschiedlichen Tiefen stammen. Werden diese Spektren entsprechend gewichtet und voneinander subtrahiert, so erhält man die Konzentration bestimmter Stoffe in Abhängigkeit von der Tiefendifferenz.

Das heißt, zu jeder Konzentrationsänderung gehört auf einer definierten Wellenlänge, welche nur innerhalb eines bestimmten Wellenlängenbereiches, der für die Substanz charakteristisch ist, eine definierte Intensitätsänderung. Unter der Annahme, dass die optischen Eigenschaften des den Stoff umgebenden Gewebes konstant sind, kann unmittelbar aus den Intensitäts- und den Verteilungsdifferenzen der zugehörige Lichtweg bestimmt werden. Einer Verteilungsdifferenz entspricht damit in vorteilhafter Weise eine bestimmte Konzentrationsdifferenz. Aus der zur Konzentrationsdifferenz gehörenden Lichtintensitätsdifferenz, kann nach dem Lambert-Beerschen Gesetz, der Weg bestimmt werden.

Die Annahme von konstanten optischen Eigenschaften des Gewebes im Messvolumen wurde vorrangehend nur zur Vereinfachung angenommen. Durch entsprechend differenzierte Algorithmen können auch Veränderungen der optischen Eigenschaften des den Stoff umgebenden Gewebes mit berücksichtigt werden, wie dies in der Praxis häufig zutrifft.

Durch das erfindungsgemäße Verfahren wird es möglich, im Stratum Corneum, in der Epidermis und bis in die Dermis hinein, Substanzen qualitativ auch bei inhomogener Verteilung in einem Messvolumen von etwa 10 µm Dicke zu bestimmen. Damit ist diese Methode geeignet, in definierte Eindringtiefe quantitative Aussagen über die Konzentration einer Substanz zu machen. Die Tiefenauflösung beträgt vorzugsweise ca. 8 µm. Hierdurch wird es möglich, die Haut in Schichtdicken von 8 µm von außen nach innen hinein, quantitativ und nicht-invasiv spektroskopisch zu untersuchen.

Die Durchführung des erfindungsgemäßen Verfahrens erfolgt gemäß der Erfindung durch ein Spektral-Photometer, dessen spektrale Auflösung 0,33nm beträgt. Dadurch wird es möglich, je nm 3 Messungen durchzuführen, so dass jedes Spektrum in sehr kleinen Abständen aufgelöst und berechnet werden kann. Hierdurch wird es möglich, Substanzen mit ähnlichen Spektren voneinander zu unterscheiden. Diese hohe Auflösung kann in vorteilhafter Weise durch ein CCD-Array mit 2048 Zellen erreicht werden.

Die Generierung, des auf die zu untersuchende Person abgestrahlten Lichtes, erfolgt vorzugsweise unter Verwendung einer Deuterium-Lampe und einer Halogenlampe, die ausreichend Licht im Bereich von 200 bis 800nm zur Verfügung stellen. Es hat sich gezeigt, dass die in-vivo vorkommenden Drogenmengen im Gewebe etwa 0,05% der Gesamtabsorption betragen. Die Intensitätsschwankungen des emittierten Lichtes der Lampe sollten daher erheblich kleiner sein als die Absorptionsänderungen durch die Drogen. Die Spannungs- und Stromstabilität der Lampe beträgt vorzugsweise weniger als 6 x 10⁻⁶ (p-p) und die Strom- und Spannungsdrift ist vorzugsweise kleiner als 0,01% pro Stunde.

Um die Lichtintensität zu verändern, kann in vorteilhafter Weise ein Abschwächelement verwendet werden, das mit Hilfe einer mechanisch verstellbaren Blende die Intensität des einfallenden Lichtes kontinuierlich verändern kann. Über einen SMA-Stecker wird die Lichtquelle über ein Lichtleiterkabel mit dem Abschwächer verbunden. Durch eine Optik kann paralleles Licht auf den Ausgang des Abschwächers projiziert werden, der ebenfalls von einem SMA-Stecker gebildet wird. Von hier aus gelangt das Licht über einen Lichtleiter zum Objekt. Zwischen den beiden SMA-Steckern befindet sich vorzugsweise eine Blende, die mit einem Schrittmotor verstellt werden kann. Die Blendenöffnung ist vorzugsweise derart berechnet, dass beim Abblenden die Lichtcharakteristik nicht wesentlich beeinflußt wird. Der Schrittmotor wird vorzugsweise von einem Rechner gesteuert, in dem sich auch die optische Aufnahmeeinheit befindet. Der Motor ist vorzugsweise derart ausgebildet, dass dieser 10.000 Schritte pro Umdrehung durchführen kann. Damit wird eine hinreichend feinstufige Veränderung der Lichtintensität möglich, um eine ausreichende Ortsauflösung in unterschiedlichen Tiefen zu erreichen.

Alternativ zu der vorangehend beschriebenen Maßnahme ist es auch möglich, durch den Einsatz von LED-Strahlern definierte Änderungen der Lichtintensität zu erreichen ohne dass sich hierbei die Abstrahleigenschaften des emittierten Lichtes verändern. Die vorrangehend beschriebene, durch beispielsweise einen Schrittmotor erreichte Ansteuerung der Blende kann hierbei entfallen.

Für die Durchführung des erfindungsgemäßen Verfahrens durch eine Auflichtmessung an der menschlichen Haut wurde ein Sensorkopf entwickelt. Dieser besitzt vorzugsweise 19 Lichtleiter, die das Licht auf die Haut bringen. 4 Lichtleiter sammeln das reflektierte Licht und leiten es zu einem CCD-Array. Eine weitere Steigerung der Qualität der Meßwerte kann erreicht werden, indem ein Lichtleiter verwendet wird, der eine nochmals größere Zahl von Lichtleitern, beispielsweise 70 Lichtleiteradern umfasst, wodurch eine noch homogenere Beleuchtung des Gewebes ermöglicht wird.

Illegale Drogen, Psychopharmaka (Neuroleptika, Antidepressiva, Sedativa und Hypnotika) Antiepileptika und Antibiotika können durch das erfindungsgemäße Verfahren nachgewiesen werden, da sich gezeigt hat, dass insbesondere in einem Wellenlängenbereich von 200 bis 800nm eine Vielzahl dieser Substanzen eine deutliche Veränderung des in-vivo gewonnenen Remissionslichtspektrums verursacht. Auf Grundlage von vorzugsweise hinsichtlich der nachzuweisenden Substanzen generierten Reinspektren sowie unter Berücksichtigung deren Lösungsverhaltens, durch Brücksichtigung des Einflusses der Lösungsmittel auf etwaige Verschiebungen der Remissionslichtspektren, durch Berücksichtigung von Photoeffekten, spektroskopisch relevanten Interaktionen zwischen den jeweiligen Substanzen, deren Absorptionseigenschaften in verschieden physiologischen Medien und ihren transkutanen Messeigenschaften wird es möglich, die Aussagefähigkeit des Analyseergebnisses noch weiter zu steigern.

### Nachweis von Methadon/Polamidon

Methadon kommt in zwei stereo-enantiomeren Formen vor. Die eine Molekülform- im folgenden D-Methadon genannt- dreht polarisiertes Licht nach rechts, die andere - im folgenden L-Polamidon genannt - nach links. Die analgetisch und entzugskompensatorisch wirksame Form ist vorwiegend die L-Form. Da in der medizinisch/therapeutischen Praxis sowohl ein Gemisch aus beiden wie auch das L-Polamidon als Reinform vorkommen, ist es wichtig, die beiden Methadone unterscheiden zu können.

Fig. 22 zeigt, dass die Spektren von D-Methadon, L-Methadon und dem Gemisch aus beiden, zum Teil sehr ähnlich und in anderen Spektralbereichen deutlich verschieden sind. Ihre Verschiedenheit erlaubt die eindeutige Diskriminierung der Enantiomere.

### Das erfindungsgemäße Verfahren kann wie folgt durchgeführt werden:

Zur Untersuchung einer Person wird auf deren Hautfläche, beispielsweise im Innenarmbereich ein Sensorkopf aufgesetzt, durch welchen Licht in einem Wellenlängenbereich von 240 bis 800nm in die Haut hinein eingestrahlt wird. Das hierbei aus einem bestimmten Hauttiefenabschnitt aus der Haut zurückgeworfene Licht wird über eine Lichtleitereinrichtung erfasst und einem Spektrometer zugeführt. Das Spektrum des über den Lichtleiter aufgefangenen Remissionslichtes wird aufgezeichnet. Das aufgezeichnete Spektrum wird durch eine Rechnereinrichtung einer mathematischen Auswerteprozedur unterzogen. Diese mathematischen Auswerteprozedur berücksichtigt vorzugsweise bestimmte Korrelationseigenschaften zwischen Spektren die für eine Vielzahl von potentiell relevanten Drogen und Medikamenten indikativ sind. Da das durch die Spektrometereinrichtung erfasste Remissionslichtspektrum sowohl für die Art der mit dem Licht in Wechselwirkung getretenen Substanz als auch eindeutig für deren Konzentration ist, ist es möglich an Hand der für eine Vielzahl von Substanzen vorhandenen Referenzspektren, Art und Konzentration der nachzuweisenden Substanzen aus dem Remissions- Lichtspektrum zu errechnen.

Sowohl die Lichtquelle zur Generierung des Untersuchungslichtes als auch der Sensorkopf zur Aufnahme des Remissionslichtes sind einschließlich des Spektrometers vorzugsweise als mobile Handgeräte aufgebaut. Es ist möglich, zur Auswertung des aufgezeichneten Remissionlichtspektrums dieses beispielsweise über ein mobiles Kommunikationssystem an eine zentrale Auswertungseinheit weiterzuleiten. Die Auswertung des derart weitergeleiteten Remissionslichtspektrums kann dann auf Grundlage eines umfassenden Datensatzes sowie durch eine leistungsfähige Hardware ausgewertet werden. Das derart gewonnene Auswertungsergebnis kann dann beispielsweise als SMS-Datensatz in den Bereich der Erfassungseinrichtung zurück übertragen werden.
Es ist auch möglich, Detektion und Auswertung online am Untersuchungsgerät selbst, etwa im Straßenverkehr, in Arztpraxen oder in stationären Behandlungseinrichtungen von Krankenhäusern durchzuführen. In diesem Fall erhält der Untersucher direkt die Meßergebnisse über eine angeschlossene Druckereinrichtung ausgedruckt.

Desweiteren können Sauerstoffbestimmungen intraoperativ online durchgeführt werden, ohne daß invasive blutgasanalytische Sauerstoffmessungen nötig sind.

In der Kinderheilkunde können Sauerstoff, Hämoglobin und Medikamentenspiegel bestimmt werden, ohne daß traumatisierende und technisch umständliche Blutabnahmen erforderlich sind.

In der Notfallmedizin können akute Intoxikationen von Drogen und Medikamenten in kürzester Zeit diagnostiziert und früher als bisher spezifisch behandelt werden.

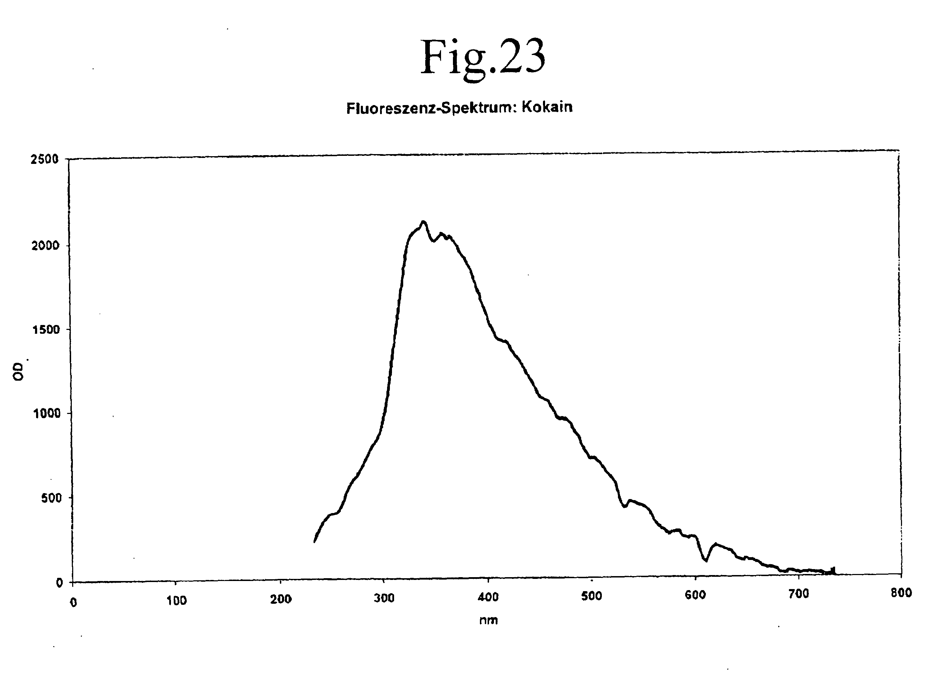

Figur 23 zeigt das Fluorezens-Spektrum von Kokain. Bei einer Wellenlänge des Anregungslichtes von 270 nm ergibt sich ein steiler Anstieg zwischen 230 und 330 nm. Im Anschluss daran fällt das Spektrum kontinuierlich ab und erreicht einen 0-Wert bei 730 nm. Bei einer Konzentration von 0,01 mg/ml ergibt sich eine optische Dichte von ca. 2080.

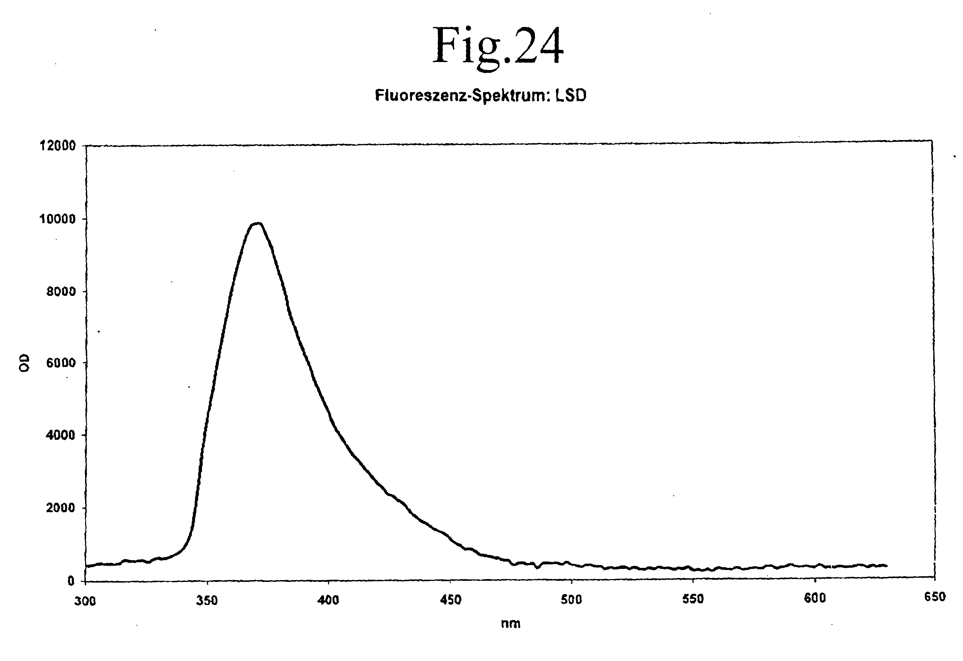

Figur 24 zeigt das Fluoreszens-Spektrum von LSD. Bei einer Wellenlänge des Anregungslichtes von 310 nm ergibt sich ein steiler Anstieg zwischen 340 und 350 nm. Danach fällt das Spektrum kontinuierlich ab und erreicht bei 450 nm wieder des Ausgangswert. Bei einer Konzentration von 0,01 mg/ml ergibt sich eine optische Dichte von ca. 10.000.

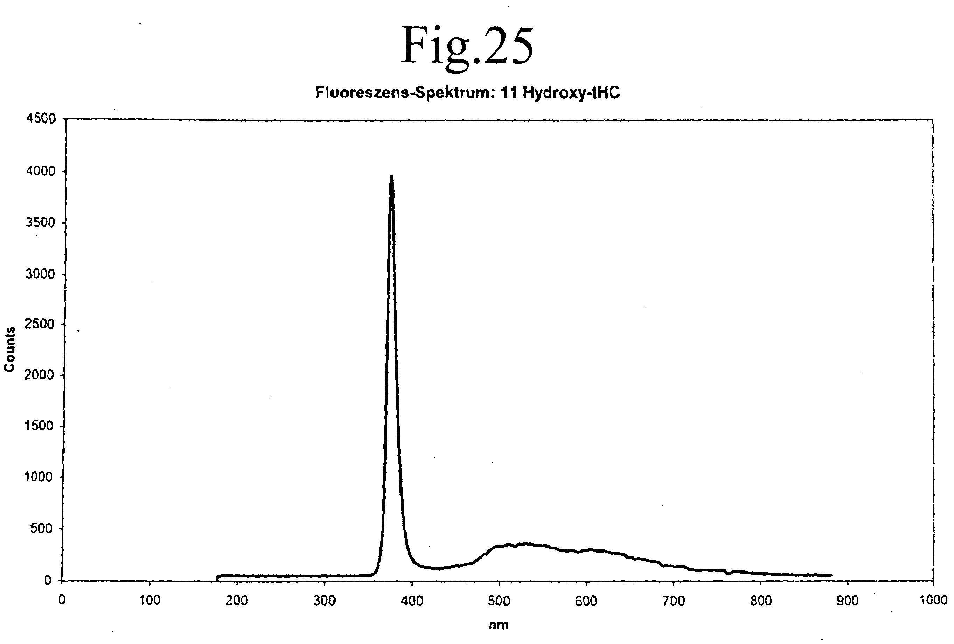

Figur 25 zeigt das Fluoreszens-Spektrum von 11 Hydroxy-THC. Bei einer Wellenlänge des Anregungslichtes von 300 nm zeigt sich zunächst ein steiler spitzer Peak zwischen 360 und 400 nm mit punctum maximum bei 390 nm. Ab 430 nm zeigt sich ein flacheres breiteres Fluoreszens-Spektrum, dass seinen Ausgangswert bei ca. 800 nm wieder erreicht. Bei einer Konzentration von 0,01 mg/ml ergibt sich eine optische Dichte in ersten Spektrum bei etwa 4100. Das zweite Spektrum erreicht den Maximalwert bei einer optischen Dichte bei etwa 490.

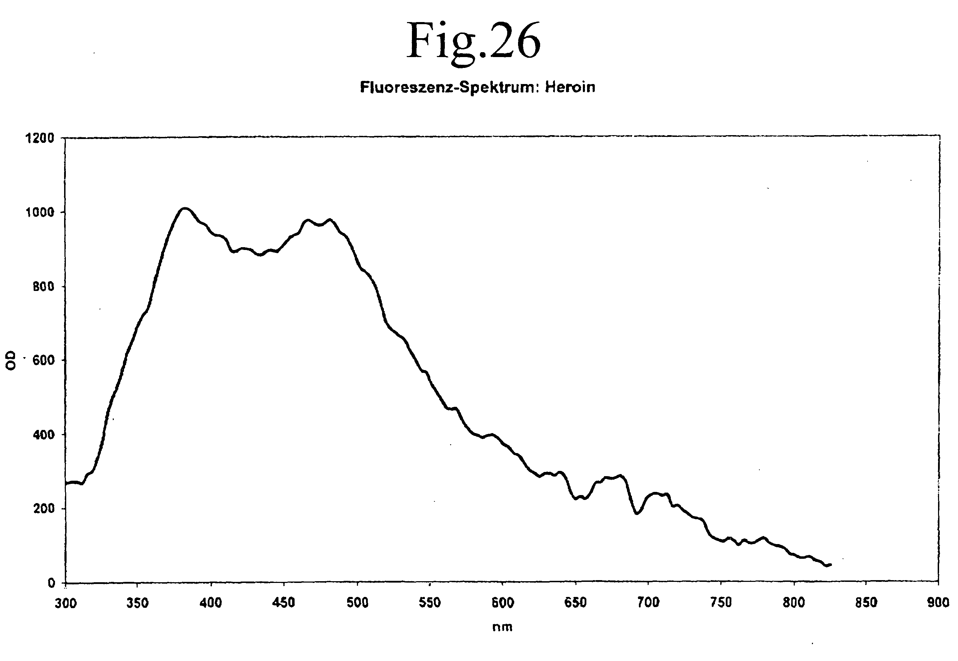

Figur 26 zeigt das Fluoreszens-Spektrum von Heroin. Bei einer Wellenlänge des Anregungslichtes von 255 nm zeigt sich ein erstes punctum maximum bei etwa 380 nm, daran anschließend eine Verringerung des Signals bei etwa 430 nm und ein zweites punctum maximum bei etwa 470 - 480 nm. Im weiteren Signalverlauf nimmt das Fluoreszens-Spektrum ab und erreicht sein punctum minimum nach inhomogenem Abfall bei etwa 420 nm.

## Patentansprüche

1. Verfahren zur Erfassung von Stoffen in vitalem Gewebe, bei welchem
- Licht mit einer vorgegebenen Wellenlänge derart auf das Gewebe gerichtet wird, dass das Licht in das Gewebe eindringt,
- zumindest ein Teil des aus dem Gewebe austretenden Lichtes erfasst wird und das remittierte Licht unter Zuordnung seiner Wellenlänge zur Intensität ermittelt wird, und
- die derart ermittelten Eigenschaften des remittierten Lichtes mit wenigstens einem Referenzsystem verglichen werden, wobei auf Grundlage einer Korrelation mit dem Referenzsystem auf die Präsenz und/oder Konzentration eines Stoffes geschlossen wird.

2. Verfahren nach Anspruch 1, **dadurch gekennzeichnet, daß** durch das Referenzsystem Referenzwerte, die jeweils für einen bestimmten Stoff oder eine Stoffgruppe signifikant sind, bereitgestellt werden und eine Intensitätsverteilung des Lichtes über seiner Wellenlänge mit diesen Referenzwerten verglichen wird.

3. Verfahren nach Anspruch 1 oder 2, **dadurch gekennzeichnet, daß** eine Vielzahl von Referenzspektren vorgegeben ist, und daß in Abhängigkeit von der Erfüllung vorgegebener Beziehungen zwischen den mehreren Referenzspektren und der erfassten Intensitätsverteilung auf die Präsenz und/oder Konzentration eines Stoffes oder einer Stoffgruppe geschlossen wird.

4. Verfahren nach wenigstens einem der Ansprüche 1 bis 3, **dadurch gekennzeichnet, daß** auf Grundlage der Intensität ausgewählter Wellenlängenbereiche auf die Konzentration eines erkannten Stoffes geschlossen wird.

5. Verfahren nach wenigstens einem der Ansprüche 1 bis 4, **dadurch gekennzeichnet, daß** das Anregungsspektrum im Bereich von 200 bis 800nm liegt.

6. Verfahren nach wenigstens einem der Ansprüche 1 bis 5, **dadurch gekennzeichnet, daß** die Frequenz des zur Anregung herangezogenen Anregungs-Lichtes außerhalb des erfaßten Remissionsspektrums liegt.

7. Verfahren nach wenigstens einem der Ansprüche 1 bis 6, **dadurch gekennzeichnet, daß** der Vergleich der erfassten Wellenlängen/Instensitäts-Verteilung auf Grundlage einer Korrelationsbetrachtung erfolgt.

8. Vorrichtung zur Erfassung von Stoffen in vitalem Gewebe, mit einer Lichtquelle zur Erzeugung von Licht definierter Wellenlänge und Aufstrahlung des Lichtes auf das vitale Gewebe derart, daß das Licht in das Gewebe eindringt, einer Lichterfassungseinrichtung zur Erfassung zumindest eines Teiles des aus dem Gewebe remittierten/reflektierten Lichtes, einer Lichtintensitätserfassungseinrichtung zur Erfassung der Intensität des remittierten/reflektierten Lichtes unter Zuordnung zur Wellenlänge, und einer Korrelationsbestimmungseinrichtung zum Bestimmen von Korrelationsmerkmalen der ermittelten Intensitätsverteilung des Remissions-Spektrums mit wenigstens einem Referenzsystem, wobei auf Grundlage der ermittelten Korrelationsmerkmale auf die Präsenz und/oder Konzentration eines Stoffes geschlossen wird.

9. Vorrichtung nach Anspruch 8, **dadurch gekennzeichnet, daß** eine Lichtleitereinrichtung vorgesehen ist, zur Aufleitung des durch die Lichtquelle emittierten Lichtes auf das Gewebe.

10. Vorrichtung nach Anspruch 8 oder 9, **dadurch gekennzeichnet, dass** eine Lichtleitereinrichtung vorgesehen ist, zur Ableitung des remittierten Lichtes.

11. Vorrichtung nach wenigstens einem der Ansprüche 8 bis 10, **dadurch gekennzeichnet, daß** eine Spektralzerlegungseinrichtung vorgesehen ist, zur Aufspaltung des remittierten Lichtes in seine Spektralanteile.

12. Vorrichtung nach Anspruch 11, **dadurch gekennzeichnet, daß** eine CCD-Aufnahmeeinrichtung vorgesehen ist, zur Bestimmung der Intensität der einzelnen Spektralanteile.

13. Vorrichtung nach Anspruch 8, **dadurch gekennzeichnet, daß** eine Einrichtung vorgesehen ist, zur Erfassung des Lichtes aus einer vorgegebenen Messtiefe.

14. Vorrichtung nach wenigstens einem der Ansprüche 8 bis 13, **dadurch gekennzeichnet, daß** mehrere Remissionsspektren für unterschiedliche, definierte Meßtiefen erfaßt werden.

15. Vorrichtung nach wenigstens einem der Ansprüche 8 bis Anspruch 14, **dadurch gekennzeichnet, daß** eine Speichereinrichtung vorgesehen ist, und daß in dieser Speichereinrichtung ein Referenzsystem in Form eines Bezugsdatensatzes abgespeichert ist.

16. Vorrichtung nach Anspruch 8, **dadurch gekennzeichnet, daß** der Bezugsdatensatz bezüglich der substanzspezifischen Spektren charakteristische Daten enthält.

17. Vorrichtung nach wenigstens einem der Ansprüche 8 bis 16, **dadurch gekennzeichnet, daß** die Korrelationsbestimmungseinrichtung mehrere Korrellationskriterien zur Analyse der Spektren bereitstellt.
